# EUROPEAN PATENT SPECIFICATION

(11) **EP 0 626 998 B1**
(45) Date of publication and mention of the grant of the patent: **31.10.2001**
(21) Application number: 93906130.5
(22) Date of filing: 18.02.1993
(51) Int. Cl.: C12N 5/10, A01N 63/02

(54) **PRODUCTION OF VIRAL RESISTANT PLANTS VIA INTRODUCTION OF UNTRANSLATABLE PLUS SENSE VIRAL RNA**
PRODUKTION VIRUS-RESISTENTER PFLANZEN DURCH EINFÜHRUNG VON NICHT-TRANSLATIERBARER VIRALER PLUS-STRANG-RNA
PRODUCTION DE PLANTES RESISTANT A DES VIRUS PAR INTRODUCTION D'ARN VIRAL DE NON TRANSLATION A SENS POSITIF

(30) Priority: 19.02.1992 US 838509
(43) Date of publication of application: 07.12.1994
(73) Proprietor: THE STATE OF OREGON ACTING BY AND THROUGH THE OREGON STATEBOARD OF HIGHER EDUCATION ON BEHALF OF THE UNIVERSITY OF OREGON, Corvallis, OR 97331-2140 (US)
(72) Inventor: DOUGHERTY, William, G., Philomath, OR 97370 (US); LINDBO, John, A., Corvallis, OR 97330 (US)
(74) Representative: Gowshall, Jonathan Vallance
(86) International application number: PCT/US93/01544
(87) International publication number: WO 93/17098

(56) References cited:
- EP-A- 0 242 016
- EP-A- 0 298 918
- EP-A- 0 325 066
- EP-A- 0 425 004
- EP-A- 0 426 195
- EP-A- 0 504 869
- WO-A-92/13089
- WO-A-92/13090
- PROCEEDINGS OF THE NATIONAL ACADEMY OF SCIENCES OF USA, vol. 87, August 1990 WASHINGTON US, pages 6291-6295, STANLEY, J., ET AL. 'Defective viral DNA ameliorates symptoms of geminivirus infection in transgenic plants'
- VIROLOGY, vol. 164, 1988 pages 383-389, VAN DUN, C.M.P., ET AL. 'Transgenic tobacco expressing tobacco streak virus or mutated alfalfa mosaic virus coat protein does not cross-protect against alfalfa mosaic virus infection'
- ANNUAL MEETING OF THE AMERICAN PHYTOPATHOLOGICAL SOCIETY, ST. LOUIS, MISSOURI, USA, AUGUST 17-21, 1991. PHYTOPATHOLOGY 81 (10). 1991. 1174. , LINDBO J A ET AL 'TRANSGENIC NICOTIANA-TABACUM PLANTS EXPRESSING VARIOUS FORMS OF THE TOBACCO ETCH VIRUS TEV COAT PROTEIN ARE RESISTANT TO TEV INFECTION.'
- Molecular Plant-Microbe Interactions, Volume 5, No. 2, issued March 1992, LINDBO et al.: "Pathogen-Derived Resistance to a Potyvirus: Immune and Resistant Phenotypes in Transgenic Tobacco Expressing Altered Forms of a Potyvirus Coat Protein Nucleotide Sequence", pages 144-153, see entire document.
- Virology, Volume 189, No. 2, issued August 1992, LINDBO et al.: "Untranslatable Transcripts of the Tobacco Etch Virus Coat Protein Gene Sequence can Interfere with Tobacco Etch Virus Replication in Transgenic Plants and Protoplasts", pages 725-733, see entire document.
- Molecular Plant-Microbe Interactions, Volume 4, No. 3, issued May 1991, KAWCHUK et al.: "Sense and Antisense RNA-Mediated Resistance to Potato Leafroll Virus in Russet Burbank Potato Plants", pages 247-253, see entire document.
- Plant Molecular Biology, Volume 17, issued 1991, VAN DER WILK et al.: "Expression of the Potato Leafroll Luteovirus Coat Protein Gene in Transgenic Potato Plants Inhibits Viral Infection", pages 431-439, see entire document.
- Journal of General Virology, Volume 72, issued August 1991, MARSH et al.: "Artificial Defective Interfering RNAs Derived from Brome Mosaic Virus", pages 1787-1792, see entire document.
- Proceedings of the National Academy of Sciences USA, Volume 88, issued August 1991, DAY et al.: "Expression of an Antisense Viral Gene in Transgenic Tobacco Confers Resistance to the DNA Virus Tomato Golden Mosaic Virus", pages 6721-6725, see entire document.
- Virology, Volume 175, issued 1990, POWELL et al.: "Protection Against Tobacco Mosaic Virus Infection in Transgenic Plants Requires Accumulation of Coat Protein Rather than Coat Protein RNA Sequences", pages 124-130, see entire document.
- Virology, Volume 154, issued 1986, ALLISON et al.: "The Nucleotide Sequence of the Coding Region of Tobacco Etch Virus Genomic RNA: Evidence for the Synthesis of a Single Polyprotein", pages 9-20, see entire document.
- Trends in Genetics, Volume 5, No. 2, issued February 1989, BAULCOMBE: "Strategies for Virus Resistance in Plants", pages 56-60, see entire document.

### SEQUENCE LISTING

(1) GENERAL INFORMATION:
   (i) APPLICANT: William G. Dougherty and John A. Lindbo
   (ii) TITLE OF INVENTION: Production of Plants Showing Immunity to Viral Infection via Introduction of Genes Encoding Untranslatable Plus Sense RNA Molecules
   (iii) NUMBER OF SEQUENCES: 4
   (iv) CORRESPONDENCE ADDRESS:
      (A) ADDRESSEE: Richard J. Polley
      (B) STREET: One World Trade Center 121 S.W. Salmon Street, Suite 1600
      (C) CITY: Portland
      (D) STATE: Oregon
      (E) COUNTRY: United States of America
      (F) ZIP: 97204
   (v) COMPUTER READABLE FORM:
      (A) MEDIUM TYPE: Diskette, 5.25 inch
      (B) COMPUTER: IBM PC Compatible
      (C) OPERATING SYSTEM: MS DOS
      (D) SOFTWARE: WordPerfect 5.1
   (vi) CURRENT APPLICATION DATA:
      (A) APPLICATION NUMBER: 07/838,509
      (B) FILING DATE: February 19, 1992
      (C) CLASSIFICATION: 435
   (vi) PRIOR APPLICATION DATA: None
   (vii) ATTORNEY/AGENT INFORMATION
      (A) NAME: Richard J. Polley, Esq.
      (B) REGISTRATION NUMBER: 28,107
      (C) REFERENCE/DOCKET NUMBER: 245-35829/RJP
   (viii) TELECOMMUNICATION INFORMATION:
      (A) TELEPHONE: (503) 226-7391
      (B) TELEFAX: (503) 228-9446
(2) INFORMATION FOR SEQ ID NO: 1:
   (i) SEQUENCE CHARACTERISTICS:
      (A) LENGTH: 9495
      (B) TYPE: Nucleic Acid
      (C) STRANDEDNESS: Single
      (D) TOPOLOGY: Linear
   (ii) MOLECULE TYPE:
      (A) DESCRIPTION: cDNA to genomic RNA
   (iii) HYPOTHETICAL: No
   (iv) ANTI-SENSE: No
   (v) FRAGMENT TYPE: N/A
   (vi) ORIGINAL SOURCE:
      (A) ORGANISM: Tobacco Etch Virus (TEV)
      (B) STRAIN: Highly Aphid Transmitted (HAT)
   (vii) IMMEDIATE SOURCE: TEV propagated in N. tabacum Burley 49
   (viii) POSITION IN GENOME: N/A
   (ix) FEATURE:
      (A) NAME/KEY: Coat protein gene
      (B) LOCATION: Genomic nucleotides 8518-9306
      (C) IDENTIFICATION METHOD: --
      (D) OTHER INFORMATION: SEQ. ID No. 1 is the cDNA corresponding to the Tobacco Etch Virus Genome.
   (x) PUBLICATION INFORMATION:
      (A) AUTHORS: Allison et al.
      (B) TITLE: The nucleotide sequence of the coding region of Tobacco Etch Virus Genomic RNA: Evidence for the Synthesis of a Single Polyprotein
      (C) JOURNAL: Virology
      (D) VOLUME: 154
      (E) ISSUE: --
      (F) PAGES: 9-20
   (xi) SEQUENCE DESCRIPTION: SEQ ID NO: 1:
(3) INFORMATION FOR SEQ ID NO: 2:
   (i) SEQUENCE CHARACTERISTICS:
      (A) LENGTH: 792
      (B) TYPE: Nucleic Acid
      (C) STRANDEDNESS: Double
      (D) TOPOLOGY: Circular
   (ii) MOLECULE TYPE: cDNA to genomic RNA
   (iii) HYPOTHETICAL: No
   (iv) ANTI-SENSE: No
   (v) FRAGMENT TYPE: N/A
   (vi) ORIGINAL SOURCE:
      (A) ORGANISM: Tobacco Etch Virus
      (B) STRAIN: Highly Aphid Transmitted
      (C) INDIVIDUAL ISOLATE: N/A
   (vii) IMMEDIATE SOURCE:
      (A) LIBRARY: No
      (B) CLONE: pTC:FL
   (viii) POSITION IN GENOME: N/A
   (ix) FEATURE:
      (A) NAME/KEY: Mutations (AGT-ATG) introduced into nucleotides corresponding to genomic nucleotides 8518-8520 of SEQ ID No. 1, to create initiating methionine codon.
      (B) LOCATION: Nucleotides 1-3 of SEQ ID No. 2
      (C) IDENTIFICATION METHOD: --
      (D) OTHER INFORMATION: SEQ ID NO: 2 is the modified Tobacco Etch Virus coat protein gene present in pTC:FL.
   (x) PUBLICATION INFORMATION:
      (A) AUTHORS: Allison et al.
      (B) TITLE: The nucleotide sequence of the coding region of Tobacco Etch Virus Genomic RNA: Evidence for the Synthesis of a Single Polyprotein
      (C) JOURNAL: Virology
      (D) VOLUME: 154
      (E) ISSUE: --
      (F) PAGES: 9-20

      (A) AUTHORS: Lindbo and Dougherty
      (B) TITLE: Untranslatable Transcripts of the tobacco etch virus coat protein gene sequence can interfere with tobacco etch virus replication in Transgenic Plants and Protoplasts
      (C) JOURNAL: Virology
      (D) VOLUME: 189
      (E) ISSUE: --
      (F) PAGES: 725-733
   (xi) SEQUENCE DESCRIPTION: SEQ ID NO: 2:
   ATG GGC ACT       9
   Met Gly Thr
     1
   GTG GAT GCT GGT GCT GAC GCT GGT AAG AAG AAA GAT CAA AAG GAT GAT      57
   Val Asp Ala Gly Ala Asp Ala Gly Lys Lys Lys Asp Gln Lys Asp Asp
         5                  10                  15
   AAA GTC GCT GAG CAG GCT TCA AAG GAT AGG GAT GTT AAT GCT GGA ACT     105
   Lys Val Ala Glu Gln Ala Ser Lys Asp Arg Asp Val Asn Ala Gly Thr
    20                  25                  30                  35
   TCA GGA ACA TTC TCA GTT CCA CGA ATA AAT GCT ATG GCC ACA AAA CTT     153
   Ser Gly Thr Phe Ser Val Pro Arg Ile Asn Ala Met Ala Thr Lys Leu
                    40                  45                  50
   CAA TAT CCA AGG ATG AGG GGA GAG GTG GTT GTA AAC TTG AAT CAC CTT     201
   Gln Tyr Pro Arg Met Arg Gly Glu Val Val Val Asn Leu Asn His Leu
                55                  60                  65
   TTA GGA TAC AAG CCA CAG CAA ATT GAT TTG TCA AAT GCT CGA GCC ACA     249
   Leu Gly Tyr Lys Pro Gln Gln Ile Asp Leu Ser Asn Ala Arg Ala Thr
            70                  75                  80
   CAT GAG CAG TTT GCC GCG TGG CAT CAG GCA GTG ATG ACA GCC TAT GGA     297
   His Glu Gln Phe Ala Ala Trp His Gln Ala Val Met Thr Ala Tyr Gly
        85                  90                  95
   GTG AAT GAA GAG CAA ATG AAA ATA TTG CTA AAT GGA TTT ATG GTG TGG     345
   Val Asn Glu Glu Gln Met Lys Ile Leu Leu Asn Gly Phe Met Val Trp
   100                 105                 110                 115
   TGC ATA GAA AAT GGG ACT TCC CCA AAT TTG AAC GGA ACT TGG GTT ATG     393
   Cys Ile Glu Asn Gly Thr Ser Pro Asn Leu Asn Gly Thr Trp Val Met
                   120                 125                 130
   ATG GAT GGT GAG GAT CAA GTT TCA TAC CCG CTG AAA CCA ATG GTT GAA      441
   Met Asp Gly Glu Asp Gln Val Ser Tyr Pro Leu Lys Pro Met Val Glu
               135                 140                     145
   AAC GCG CAG CCA ACA CTG AGG CAA ATT ATG ACA CAC TTC AGT GAC CTG      489
   Asn Ala Gln Pro Thr Leu Arg Gln Ile Met Thr His Phe Ser Asp Leu
           150                 155                     160
   GCT GAA GCG TAT ATT GAG ATG AGG AAT AGG GAG CGA CCA TAC ATG CCT      537
   Ala Glu Ala Tyr Ile Glu Met Arg Asn Arg Glu Arg Pro Tyr Met Pro
       165                 170                     175
   AGG TAT GGT CTA CAG AGA AAC ATT ACA GAC ATG AGT TTG TCA CGC TAT      585
   Arg Tyr Gly Leu Gln Arg Asn Ile Thr Asp Met Ser Leu Ser Arg Tyr
   180                 185                     190                 195
   GCG TTC GAC TTC TAT GAG CTA ACT TCA AAA ACA CCT GTT AGA GCG AGG      633
   Ala Phe Asp Phe Tyr Glu Leu Thr Ser Lys Thr Pro Val Arg Ala Arg
                   200                     205                 210
   GAG GCG CAT ATG CAA ATG AAA GCT GCT GCA GTA CGA AAC AGT GGA ACT      681
   Glu Ala His Met Gln Met Lys Ala Ala Ala Val Arg Asn Ser Gly Thr
               215                     220                 225
   AGG TTA TTT GGT CTT GAT GGC AAC GTG GGT ACT GCA GAG GAA GAC ACT      729
   Arg Leu Phe Gly Leu Asp Gly Asn Val Gly Thr Ala Glu Glu Asp Thr
           230                 235                     240
   GAA CGG CAC ACA GCG CAC GAT GTG AAC CGT AAC ATG CAC ACA CTA TTA      777
   Glu Arg His Thr Ala His Asp Val Asn Arg Asn Met His Thr Leu Leu
       245                 250                     255
   GGG GTC CGC CAG TGA                                                  792
   Gly Val Arg Gln
   260
(4) INFORMATION FOR SEQ ID NO: 3:
   (i) SEQUENCE CHARACTERISTICS:
      (A) LENGTH: 793
      (B) TYPE: Nucleic Acid
      (C) STRANDEDNESS: Double
      (D) TOPOLOGY: Circular
   (ii) MOLECULE TYPE: cDNA to genomic RNA
   (iii) HYPOTHETICAL: No
   (iv) ANTI-SENSE: No
   (v) FRAGMENT TYPE: N/A
   (vi) ORIGINAL SOURCE:
      (A) ORGANISM: Tobacco Etch Virus
      (B) STRAIN: Highly Aphid Transmitted
      (C) INDIVIDUAL ISOLATE: N/A
   (vii) IMMEDIATE SOURCE:
      (A) LIBRARY: No
      (B) CLONE: pTC:RC
   (viii) POSITION IN GENOME: N/A
   (ix) FEATURE:
      (A) NAME/KEY: Mutation of AGT-GGC (Ser-Gly) to ATG-GCC (Met-Ser)
      (B) LOCATION: Nucleotides 1-6 of SEQ ID NO. 3 (corresponding to nucleotides 8518-8523 of SEQ ID NO. 1)
      (A) NAME/KEY: Frameshift mutation (insertion of T) producing stop codon
      (B) LOCATION: Nucleotide 13 of SEQ ID No. 3 (corresponding to position between nucleotides 8529 and 8530 of SEQ. ID No. 1)
      (D) OTHER INFORMATION: SEQ ID No: 3 is the modified Tobacco Etch Virus coat protein gene present in pTC:RC.
   (x) PUBLICATION INFORMATION:
      (A) AUTHORS: J. A. Lindbo and W. G. Dougherty
      (B) TITLE: Pathogen-Derived Resistance to a Potyvirus: Immune and Resistant Phenotypes in Transgenic Tobacco Expressing Altered Forms of a Potyvirus Coat Protein Nucleotide Sequence
      (C) JOURNAL: Molecular Plant-Microbe Interactions
      (D) VOLUME: 5
      (E) ISSUE: 2
      (F) PAGES: 144-153

      (A) AUTHORS: J. A. Lindbo and W. G. Dougherty
      (B) TITLE: Untranslatable Transcripts of the Tobacco Etch Virus Coat Protein Gene Sequence Can Interfere with Tobacco Etch Virus Replication in Transgenic Plants and Protoplasts
      (C) JOURNAL: Virology
      (D) VOLUME: 189
      (E) ISSUE: --
      (F) PAGES: 725-733
   (xi) SEQUENCE DESCRIPTION: SEQ ID NO: 3:
   ATG GCC ACT              9
   Met Ser Thr
   GTG TGA TGA TGGTGCTAGC GCTGGTAAGA AGAAAGATCA AAAGGATGAT                           58
   Val
   AAAGTCGCTG AGCAGGCTTC AAAGGATAGG GATGTTAATG CTGGAACTTC        108
   AGGAACATTC TCAGTTCCAC GAATAAATGC TATGGCCACA AAACTTCAAT        158
   ATCCAAGGAT GAGGGGAGAG GTGGTTGTAA ACTTGAATCA CCTTTTAGGA        208
   TACAAGCCAC AGCAAATTGA TTTGTCAAAT GCTCGAGCCA CACATGAGCA        258
   GTTTGCCGCG TGGCATCAGG CAGTGATGAC AGCCTATGGA GTGAATGAAG        308
   AGCAAATGAA AATATTGCTA AATGGATTTA TGGTGTGGTG CATAGAAAAT        358
   GGGACTTCCC CAAATTTGAA CGGAACTTGG GTTATGATGG ATGGTGAGGA        408
   TCAAGTTTCA TACCCGCTGA AACCAATGGT TGAAAACGCG CAGCCAACAC        458
   TGAGGCAAAT TATGACACAC TTCAGTGACC TGGCTGAAGC GTATATTGAG        508
   ATGAGGAATA GGGAGCGACC ATACATGCCT AGGTATGGTC TACAGAGAAA        558
   CATTACAGAC ATGAGTTTGT CACGCTATGC GTTCGACTTC TATGAGCTAA        608
   CTTCAAAAAC ACCTGTTAGA GCGAGGGAGG CGCATATGCA AATGAAAGCT        658
   GCTGCAGTAC GAAACAGTGG AACTAGGTTA TTTGGTCTTG ATGGCAACGT        708
   GGGTACTGCA GAGGAAGACA CTGAACGGCA CACAGCGCAC GATGTGAACC        758
   GTAACATGCA CACACTATTA GGGGTCCGCC AGTGA                        793
(5) INFORMATION FOR SEQ ID NO: 4
   (i) SEQUENCE CHARACTERISTICS:
      (A) LENGTH: 792
      (B) TYPE: Nucleic acid
      (C) STRANDEDNESS: Double
      (D) TOPOLOGY: Circular
   (ii) MOLECULE TYPE: cDNA to genomic RNA
   (iii) HYPOTHETICAL: No
   (iv) ANTI-SENSE: Yes
   (v) FRAGMENT TYPE: N/A
   (vi) ORIGINAL SOURCE:
      (A) ORGANISM: Tobacco Etch Virus
      (B) STRAIN: Highly Aphid Transmitted
      (C) INDIVIDUAL ISOLATE: N/A
   (vii) IMMEDIATE SOURCE:
      (A) LIBRARY: No
      (B) CLONE: pTC:AS
   (viii) POSITION IN GENOME: N/A
   (ix) FEATURE:
      (A) NAME/KEY: --
      (B) LOCATION: --
      (C) IDENTIFICATION METHOD: --
      (D) OTHER INFORMATION: SEQ ID No. 4 is the modified Tobacco Etch Virus Coat protein gene present in pTC:AS. It is the inverse complement of SEQ ID No. 2.
   (x) PUBLICATION INFORMATION:
      (A) AUTHORS: J. A. Lindbo and W. G. Dougherty
      (B) TITLE: Untranslatable Transcripts of the Tobacco Etch Virus Coat Protein Gene Sequence Can Interfere with Tobacco Etch Virus Replication in Transgenic Plants and Protoplasts
      (C) JOURNAL: Virology
      (D) VOLUME: 189
      (E) ISSUE: --
      (F) PAGES: 725-733

      (A) AUTHORS: J. A. Lindbo and W. G. Dougherty
      (B) TITLE: Pathogen-Derived Resistance to a Potyvirus: Immune and Resistant Phenotypes in Transgenic Tobacco Expressing Altered Forms of a Potyvirus Coat Protein Nucleotide Sequence
      (C) JOURNAL: Molecular Plant-Microbe Interactions
      (D) VOLUME: 5
      (E) ISSUE: 2
      (F) PAGES: 144-153
   (xi) SEQUENCE DESCRIPTION: SEQ ID NO: 4:
   TCACTGGCGG ACCCCTAATA GTGTGTGCAT GTTACGGTTC ACATCGTGCG CTGTGTGCCG     60
   TTCAGTGTCT TCCTCTGCAG TACCCACGTT GCCATCAAGA CCAAATAACC TAGTTCCACT    120
   GTTTCGTACT GCAGCAGCTT TCATTTGCAT ATGCGCCTCC CTCGCTCTAA CAGGTGTTTT    180
   TGAAGTTAGC TCATAGAAGT CGAACGCATA GCGTGACAAA CTCATGTCTG TAATGTTTCT    240
   CTGTAGACCA TACCTAGGCA TGTATGGTCG CTCCCTATTC CTCATCTCAA TATACGCTTC    300
   AGCCAGGTCA CTGAAGTGTG TCATAATTTG CCTCAGTGTT GGCTGCGCGT TTTCAACCAT    360
   TGGTTTCAGC GGGTATGAAA CTTGATCCTC ACCATCCATC ATAACCCAAG TTCCGTTCAA    420
   ATTTGGGGAA GTCCCATTTT CTATGCACCA CACCATAAAT CCATTTAGCA ATATTTTCAT    480
   TTGCTCTTCA TTCACTCCAT AGGCTGTCAT CACTGCCTGA TGCCACGCGG CAAACTGCTC    540
   ATGTGTGGCT CGAGCATTTG ACAAATCAAT TTGCTGTGGC TTGTATCCTA AAAGGTGATT    600
   CAAGTTTACA ACCACCTCTC CCCTCATCCT TGGATATTGA AGTTTTGTGG CCATAGCATT    660
   TATTCGTGGA ACTGAGAATG TTCCTGAAGT TCCAGCATTA ACATCCCTAT CCTTTGAAGC    720
   CTGCTCAGCG ACTTTATCAT CCTTTTGATC TTTCTTCTTA CCAGCGTCAG CACCAGCATC    780
   CACAGTGCCC AT                                                        792

## Description

### FIELD OF THE INVENTION

This invention is directed to the production of plants with a reduced susceptibility to virus infection.

### BACKGROUND OF THE INVENTION

Plant viruses are responsible for major losses in worldwide crop production. Much effort is directed towards the development of new plant varieties which exhibit increased resistance to viral infection. Until recently such efforts were primarily based on the traditional plant breeding approach, however this approach is often limited by a lack of sources of resistance within the crop species. The advent of modern molecular biology techniques has facilitated the development of new methods of rendering plant varieties resistant to virus attack that are not limited by a requirement for preexisting resistance genes within a species.

### Molecular Approaches

Many of these molecular approaches are based on the theory of pathogen derived resistance (Sanford and Johnston, 1985). This theory predicts that a "normal" host (plant) - pathogen (virus) relationship can be disrupted if the host organism expresses essential pathogen derived genes. It has been proposed that host organisms expressing pathogen gene products in excess amounts, at an inappropriate developmental stage, or in a dysfunctional form may disrupt the normal replicative cycle of the pathogen and result in an attenuated or aborted infection of the host.

Two approaches typify this pathogen derived resistance: coat protein mediated resistance and antisense RNA expression. It has been demonstrated that transgenic plants expressing a plant virus coat protein can be resistant to infection by the homologous virus. This coat protein mediated resistance has been demonstrated for several virus groups. While the mechanism of this resistance is not yet fully understood, it has been suggested that the presence of the plant synthesized coat protein prevents the removal of the protein coat (uncoating) of an invading virus and/or virus movement within the infected plant, leading to resistance.

Plants which express an RNA molecule which is complementary to plus sense RNA species encoded by the virus may show a decreased susceptibility to infection by that virus. Such a complementary RNA molecule is termed antisense RNA. It is thought that the plant encoded antisense RNA binds to the viral RNA and thus inhibits its function.

### Potyviruses

The Potato Virus Y, or potyvirus, family represents a large number of plant viral pathogens which collectively can infect most crop species including both monocotyledonous and dicotyledonous plants. Potyvirus infection can induce a variety of symptoms including leaf mottling, seed and fruit distortion and can severely compromise crop yield and/or quality (Hollings and Brunt, 1981).

Potyviruses have a single-strand plus sense RNA of circa 10,000 nucleotides which has a viral encoded protein linked to the 5' end and a 3' polyadenylate region. A single open reading frame codes for a 351 kDa polyprotein which is proteolytically processed into mature viral gene products. The RNA is encapsidated by approximately 2,000 copies of a coat protein monomer to form a virion. This capsid protein is encoded by the sequence present at the 3' end of the large open reading frame.

Potyviruses can be transmitted by aphids and other sap feeding insects and in some instances can also be transmitted in the seeds of infected plants. Replication of the viral RNA is thought to occur in the cytoplasm of infected plant cells after uncoating. The replication mechanism involves both translation of the plus sense RNA to yield viral gene products (which include a replicase and a proteinase) and also the synthesis of a minus sense RNA strand. This minus sense strand then acts as a template for the synthesis of many plus sense genomes which are subsequently encapsidated in coat protein to yield infectious mature "virions", thus complete the replicative cycle of the virus.

Experiments have been reported in which transgenic plants expressing the coat protein gene of a potyvirus show a reduced susceptibility to virus infection (Lawson *et al*. 1990; Ling *et al*. 1991; Stark and Beachy 1989).

EP-A-0242016 discloses the incorporation of genetic material, in particular cDNA corresonding to plant viral satellite RNA, into a plant such that, when the plant is infected by a plant virus, the expression of the incorporated material modifies the plant virus or its effects.

WO-A-9213090 discloses a method for producing transgenic plants with reduced virus susceptibility.

### SUMMARY OF THE INVENTION

The disclosed invention concerns a method of producing plants with a decreased susceptibility to virus infection. This is achieved by transforming plants with a DNA molecule which includes a gene derived in part from the genome of a plant virus. This gene is specifically constructed to produce an untranslatable version of a plus sense RNA molecule required for viral replication. Thus, expression of the gene within the plant causes the production of this non-functional molecule which then inhibits viral replication within the plant, rendering the plant resistant to viral infection.

In particular, invention provides an alternative and novel approach to rendering plants resistant to potyvirus infection.

Plants are transformed with a gene construct engineered to express an untranslatable form of the plus sense RNA which encodes the coat protein of a potyvirus.

In the case of Tobacco Etch Virus (TEV), it is demonstrated that tobacco plants transformed with such a gene construct accumulate the untranslatable plus sense RNA but do not produce detectable levels of the coat protein. It is further shown that these plants are resistant to TEV infection. It is also shown that tobacco cells expressing this untranslatable plus sense RNA do not support TEV replication, unlike control tobacco cells and also unlike tobacco cells which are engineered to express the plus sense translatable RNA and which, as a result, accumulate TEV coat protein. Although the exact mechanism is unknown, it is proposed that the untranslatable plus sense RNA inhibits viral replication by binding to the minus sense RNA and preventing the minus sense RNA from functioning in the replication cycle.

It is believed that this approach will be applicable to other potyviruses, to genes other than the coat protein gene and to other plus sense RNA virus families. It is also believed that this means of inhibiting gene function is applicable to other biological systems, including mammalian viruses.

### DESCRIPTION OF DRAWINGS

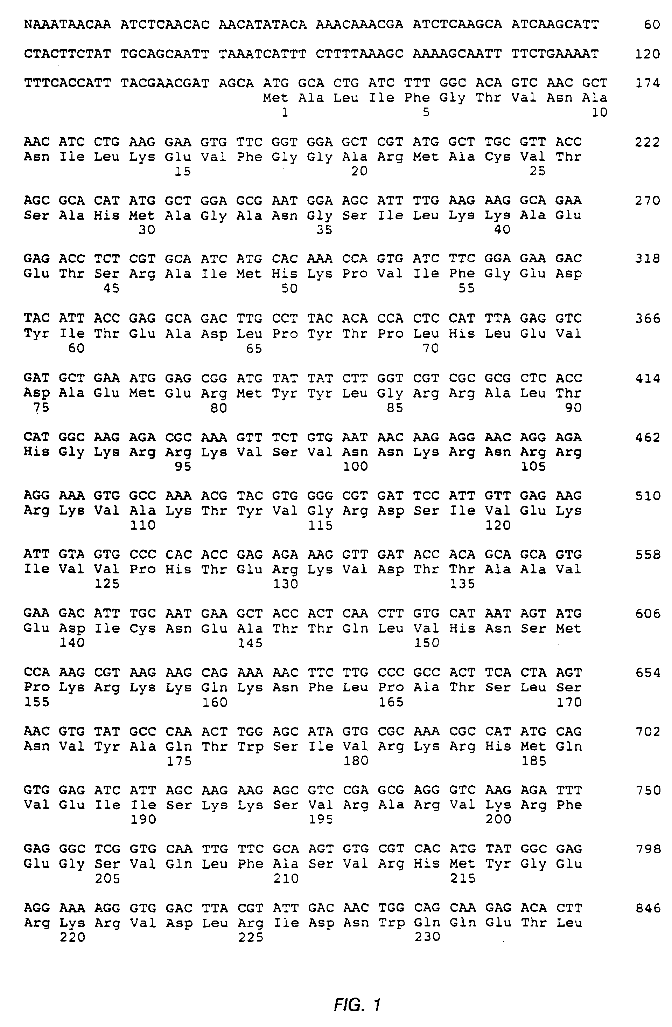

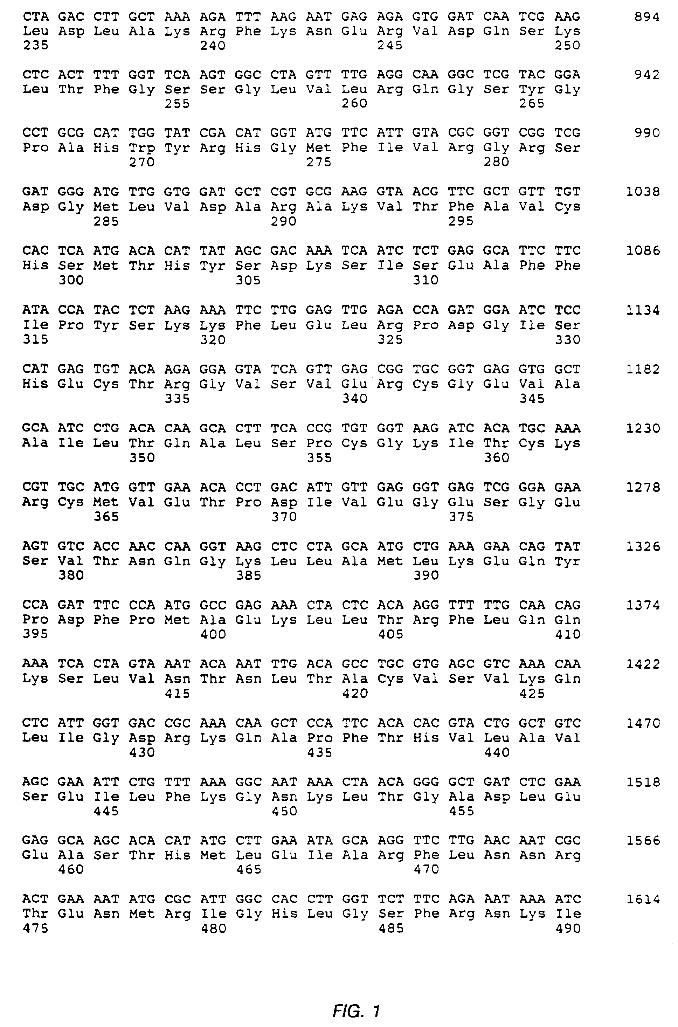

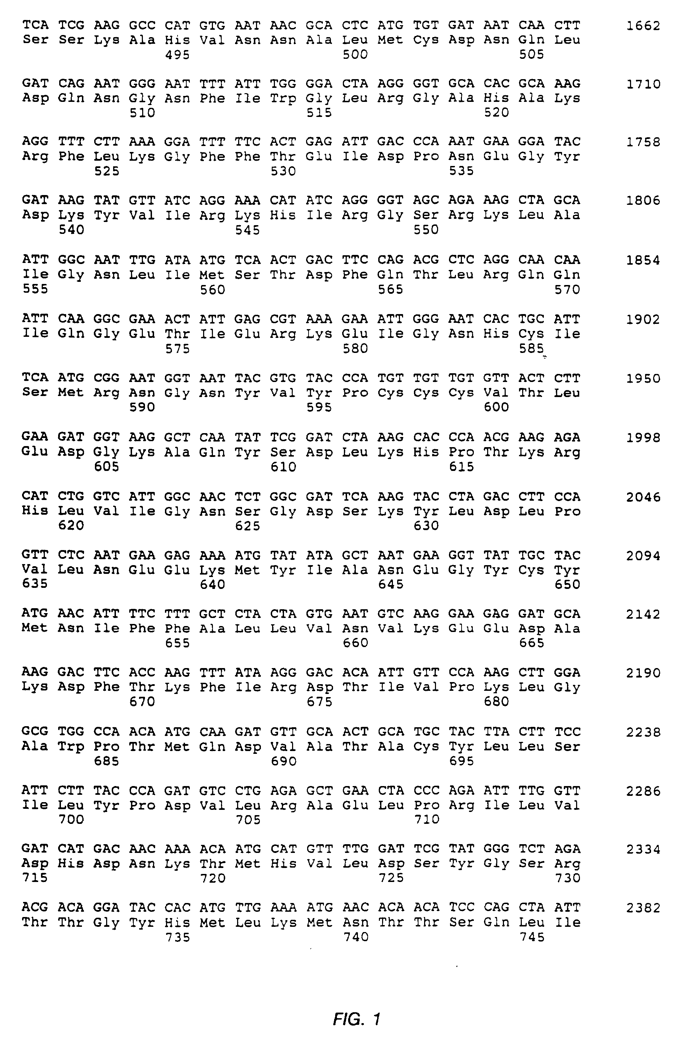

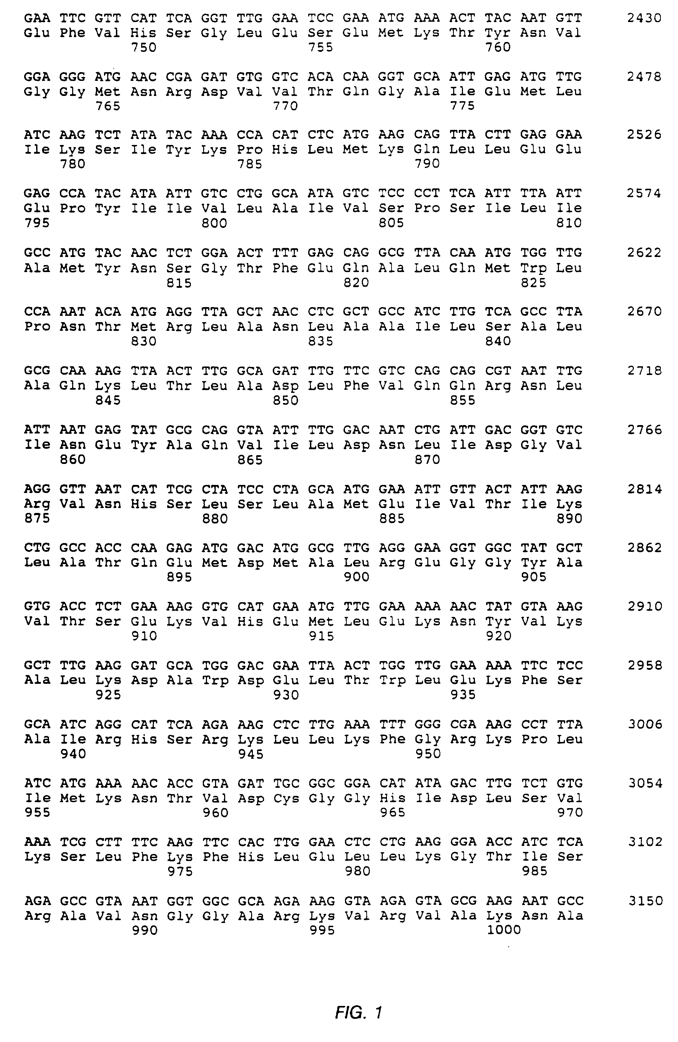

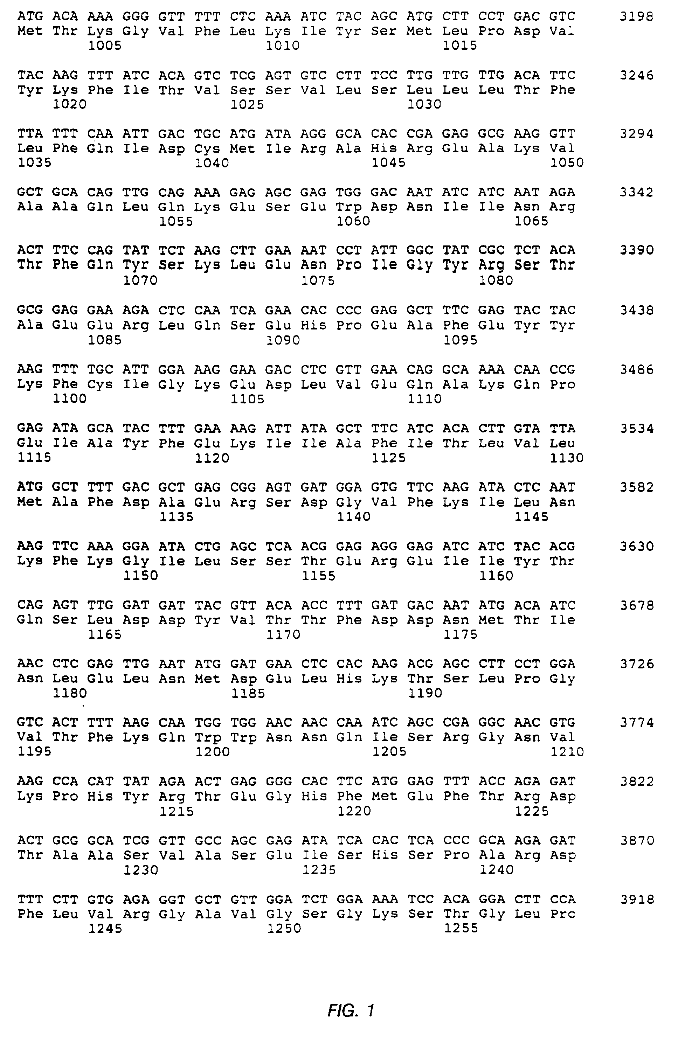

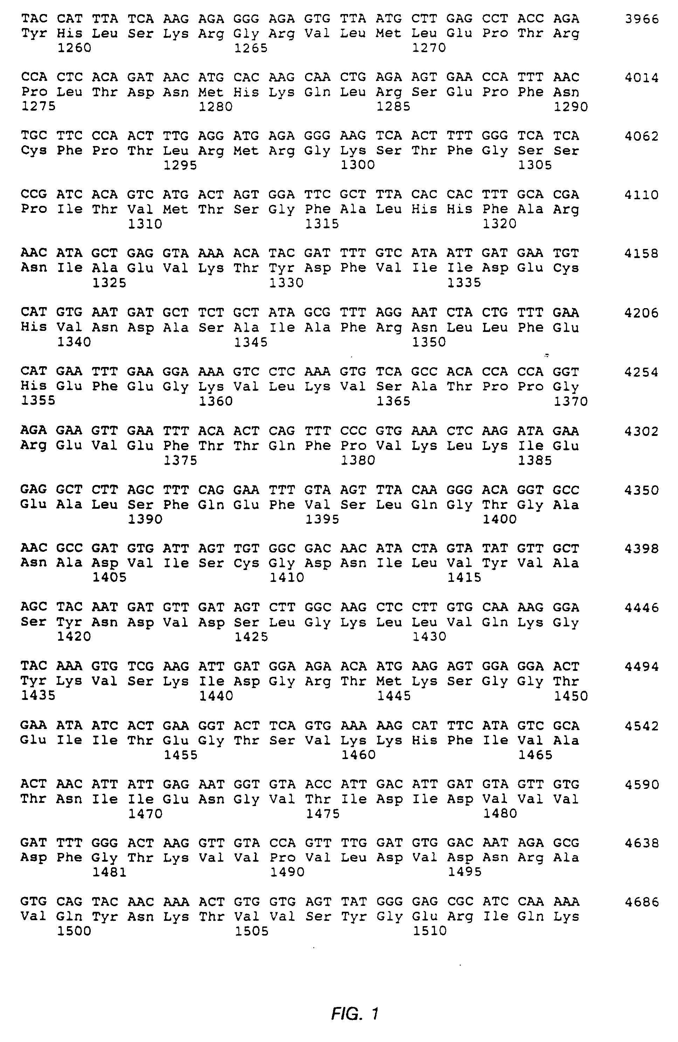

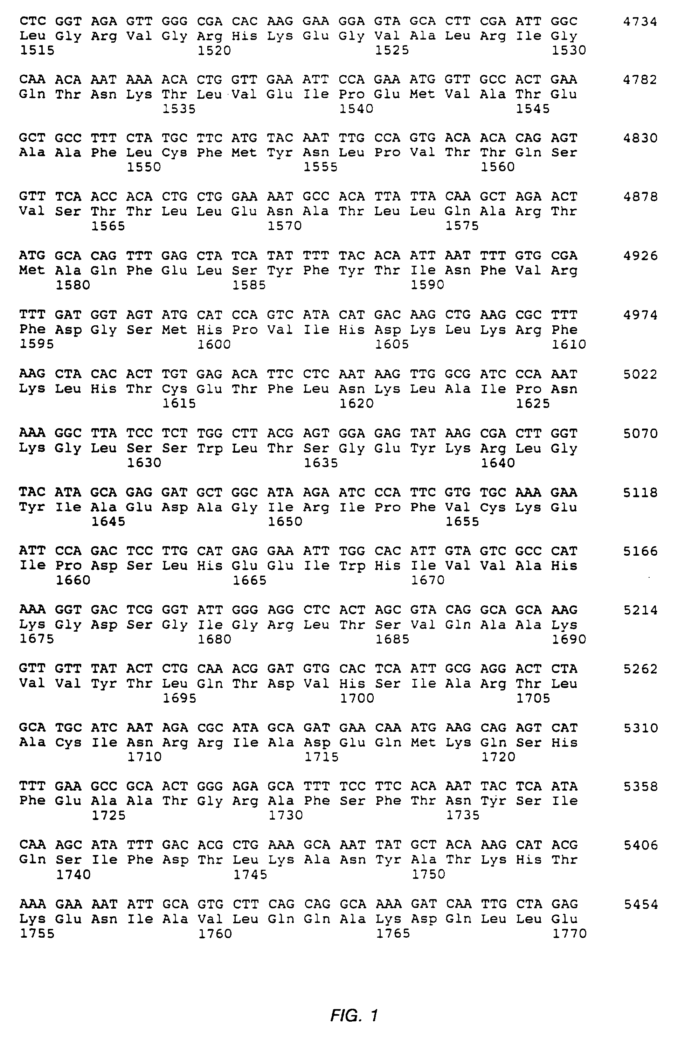

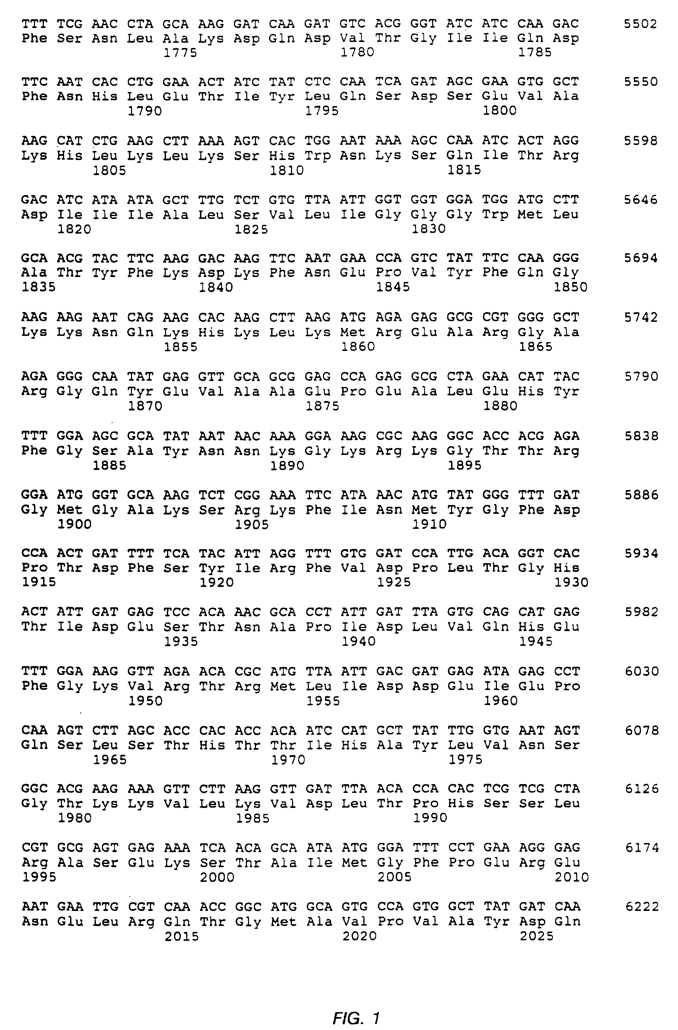

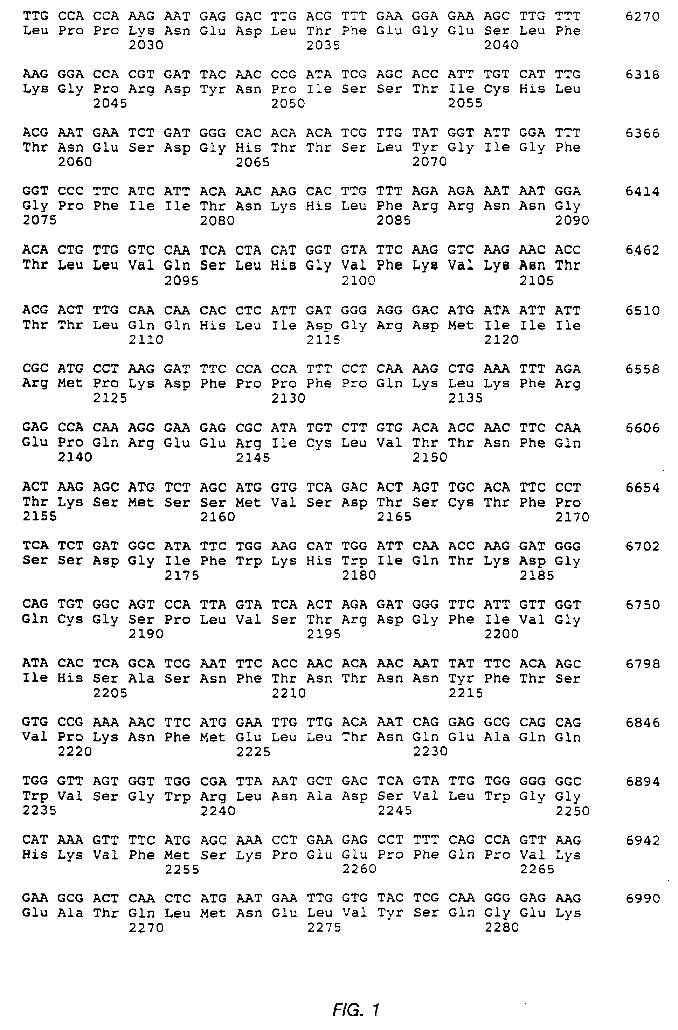

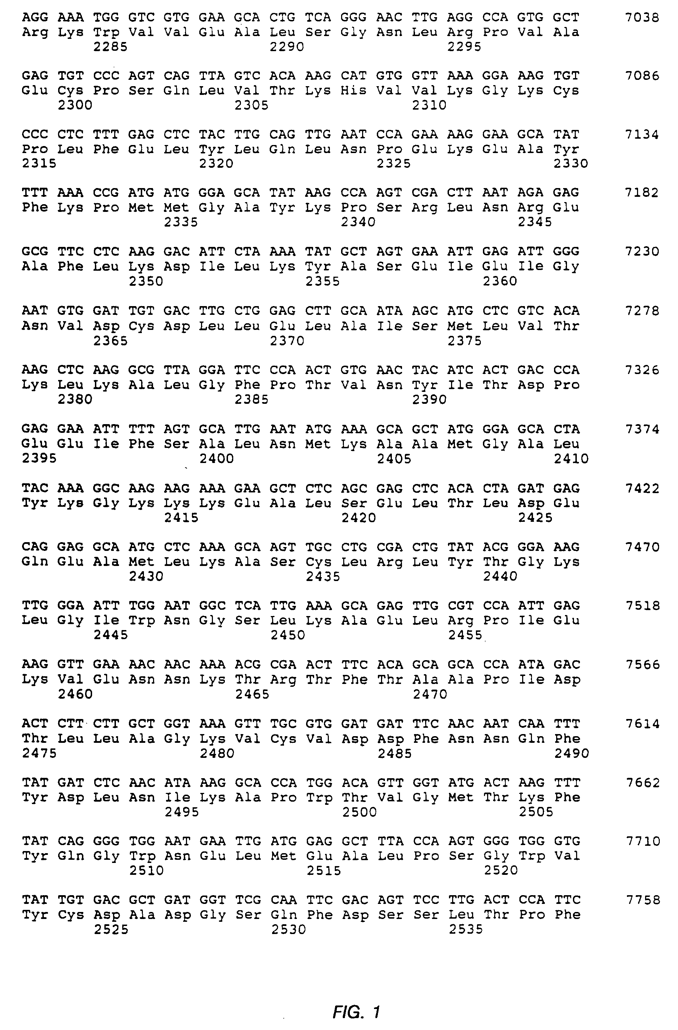

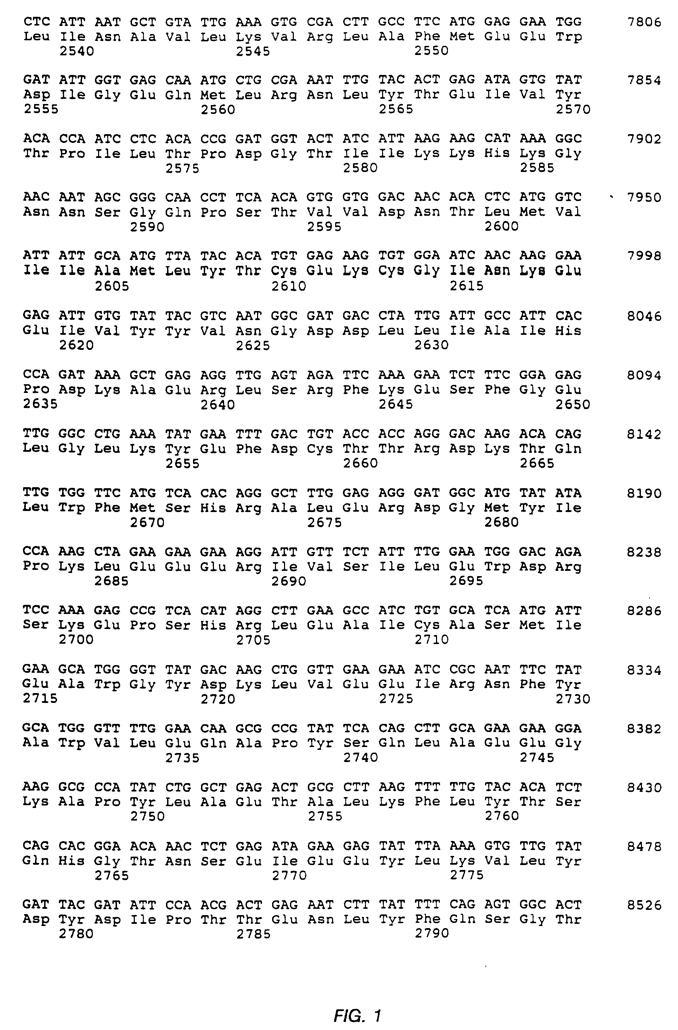

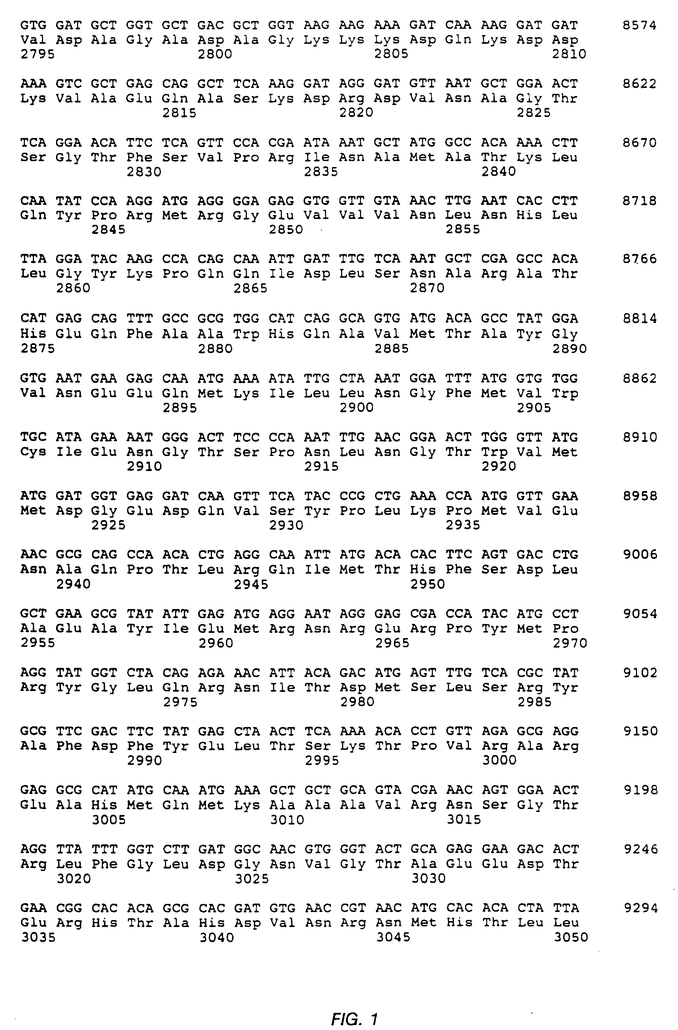

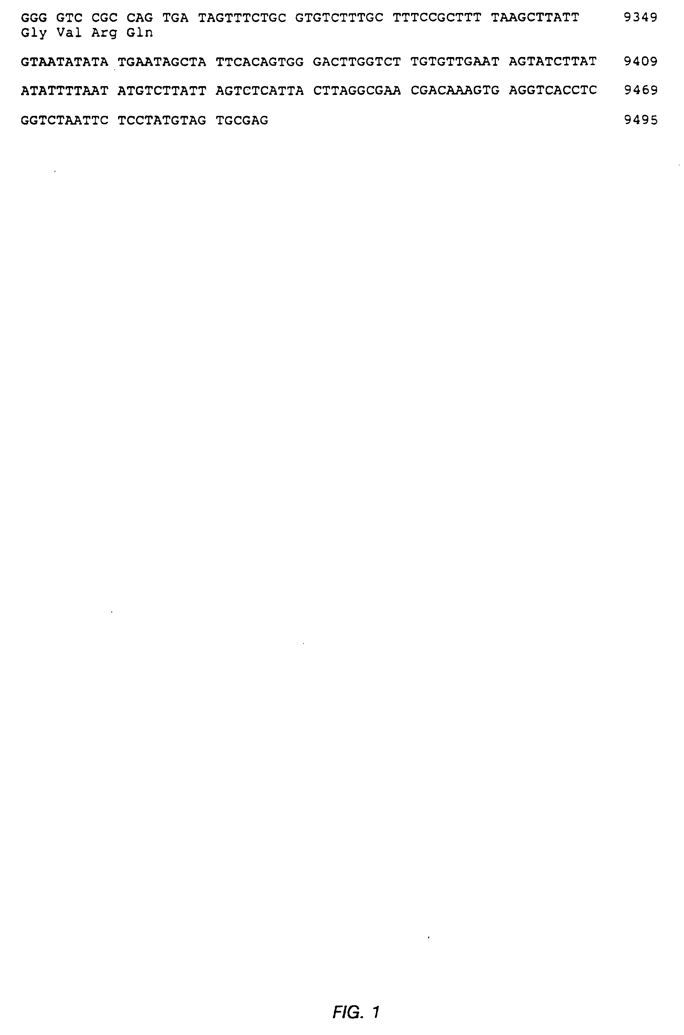

Fig. 1 represents the nucleotide sequence of the Tobacco Etch Virus genome and its deduced amino acid sequence, according to Allison et al. (1986). The nucleotide sequence of the plus sense strand of the DNA inserts is given. The first nucleotide (N) could not be determined unequivocally. The predicted amino acid sequence of the large ORF of reading frame three of the viron sense RNA is presented in the nucleotide sequence. This sequence is also set forth in SEQ ID No. 1 of the enclosed sequence listing. The termination codon at the end of the large ORF is marked with a *. The putative cleavage site between the large (54,000 *Mw)* nuclear inclusion protein and the capsid protein is indicated by the arrow. Oligonucleotide primer binding sites are underlined and labeled.

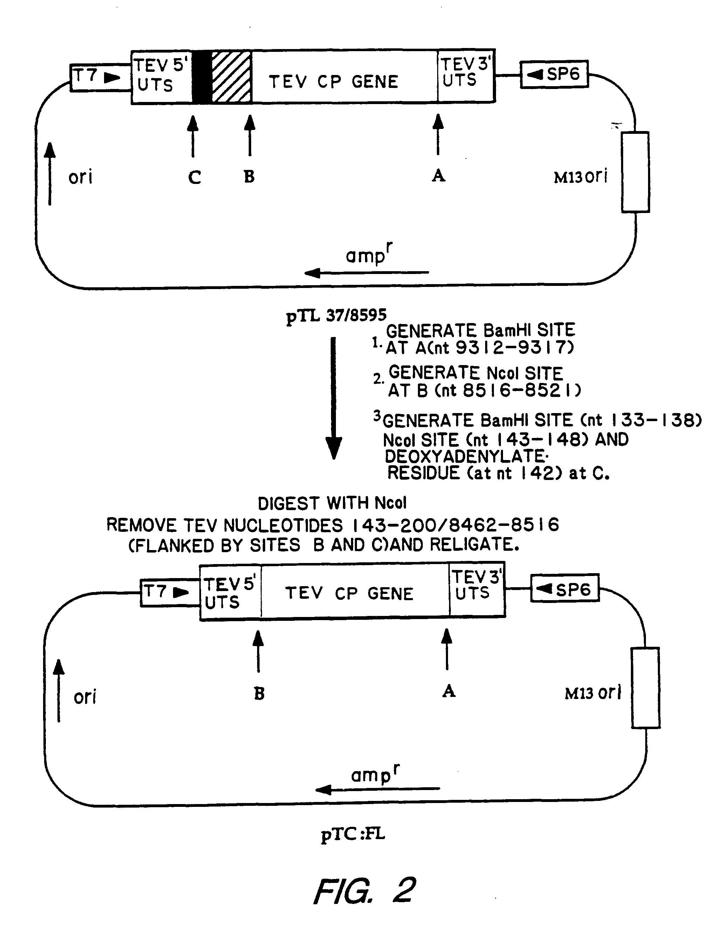

Fig. 2 is a schematic representation of the construction of pTC:FL, utilized in construction of transformation vectors for the invention. Restriction endonuclease sites were introduced into pTL 37/8595 at positions A, B and C in the diagram. Following these nucleotide changes the mutated pTL 37/8595 was digested with the restriction enzyme *NcoI,* the DNA fragment delineated by the restriction enzyme sites at B and C was removed, and the plasmid religated to generate pTC:FL. pTC:FL contains the Tobacco Etch Virus (TEV) coat protein nucleotide sequence flanked by *Bam*HI restriction sites and the TEV 5' and 3' untranslated sequences (UTS). T7 and SP6 promoters are also shown. Abbreviations used in this diagram are as follows: T7, T7 RNA polymerase promoter sequence; SP6, SP6 RNA polymerase promoter sequence; ori, origin of replication; M13 ori, bacteriophage M13 single-stranded origin of replication; amp^{r}, *β*-lactamase gene. Lightly stippled areas are TEV 5' and 3' untranslated sequences; solid black area, TEV genome cDNA nucleotides 144 to 200; striped area, a portion of the TEV NIb gene (TEV nt 8462-8517); heavily stippled areas, cDNA of TEV CP nucleotide sequence (TEV nt 8518-9309).

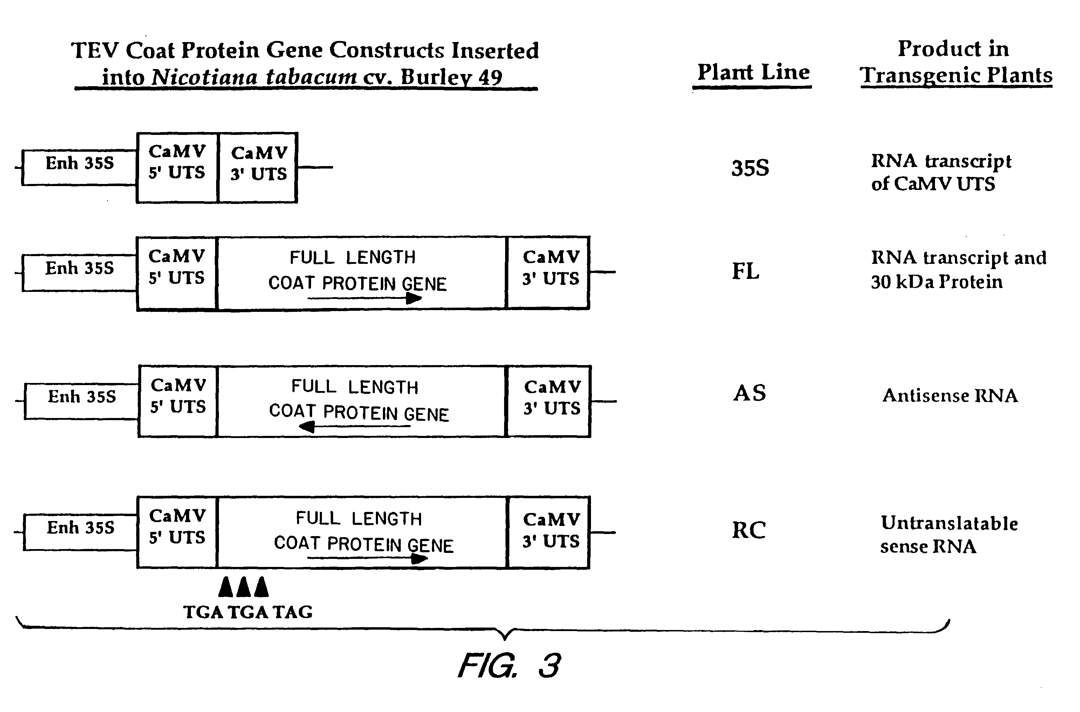

Fig. 3 is a schematic representation of the forms of the Tobacco Etch Virus coat protein gene inserted into tobacco in the invention. All constructs contained the enhanced CaMV 35S (Enh 35S) promoter, CaMV 35S 5' untranslated sequence (UTS) of 50 bp and the CaMV 35S 3' UTS/polyadenylation site of 110 bp. The nomenclature used to describe the transgenic plant lines is presented along with the gene products produced in those plant lines (far right column). Abbreviations are as follows: 35S, transgenic plants containing the CaMV 35S promoter and 5' and 3' UTS only; FL, transgenic plants containing the transgene coding for full-length, AS and RC transgenic plants contain the transgene expressed as an antisense form of the TEV CP gene, or an untranslated sense form of the TEV CP gene, respectively. Stippled areas represent various forms of the TEV CP nucleotide sequence.

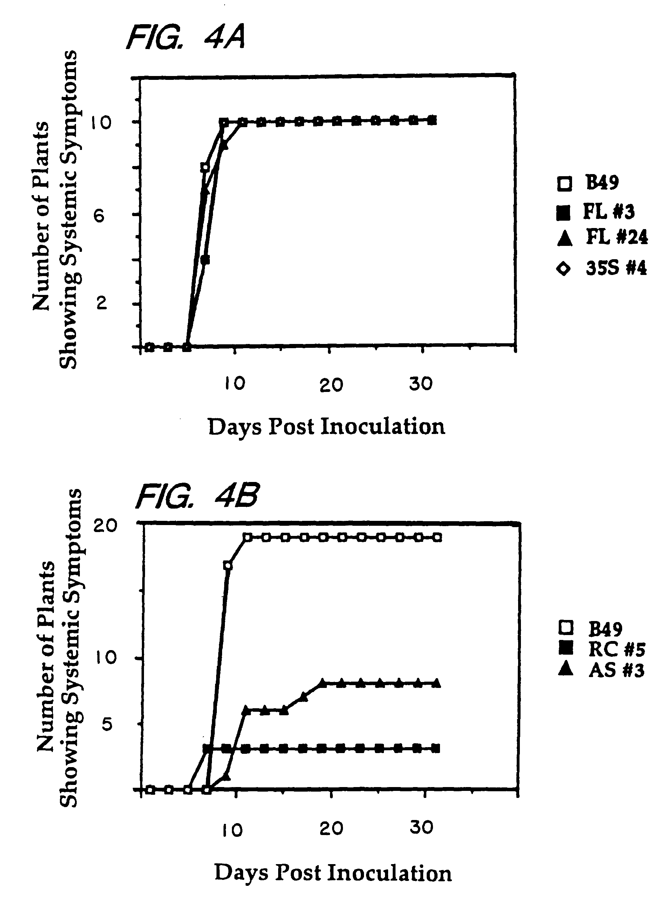

Fig. 4 is a graphic representation of the appearance of systemic symptoms in plants infected with Tobacco Etch Virus showing responses of control plants and transformed plants generated as described in the invention. Ten B49 (wild type) plants and ten R2 plants of transgenic plant lines 35S #4, FL #3, FL #24, homozygous for the inserted TEV gene, were mechanically inoculated with 50 *µ*l of 1:10 dilution of infected plant sap (A). Twenty B49 plants and 20 R1 plants of lines AS #3 and RC #5 were mechanically inoculated with 50 *µ*l of 5 *µ*g/ml TEV (B). Plants were examined daily for the appearance of systemic symptoms. Plants were evaluated daily, and any plant displaying systemic symptoms (attenuated or wild-type) were recorded as symptomatic.

### SEQUENCE LISTING

The attached sequence listing sets forth nucleotide sequences relevant to the present invention.

SEQ ID No. 1 is the complementary DNA sequence corresponding to the Tobacco Etch Virus Genome.

SEQ ID No. 2 is the nucleotide sequence of the modified Tobacco Etch Virus coat protein gene present in pTC:FL.

SEQ ID No. 3 is the nucleotide sequence of the modified Tobacco Etch Virus coat protein gene present in pTC:RC.

SEQ ID No. 4 is the nucleotide sequence of the modified Tobacco Etch Virus coat protein gene present in pTC:AS. It is the inverse complement of SEQ ID No. 2.

### DETAILED DESCRIPTION

The present invention relates to genetically engineered plants which are transformed with a DNA molecule encoding an untranslatable plus sense RNA molecule.

### Definition of Terms

Susceptible plant: A plant that supports viral replication and displays virus-induced symptoms.

Resistant plant: A plant wherein virus-induced symptoms are attenuated and virus replication is attenuated.

Plus sense RNA (and sense RNA): That form of an RNA which can serve as messenger RNA.

Minus sense RNA: That form of RNA used as a template for plus sense RNA production.

Antisense RNA: RNA complementary to plus sense RNA form.

Rₒ generation: Primary transformants.

R₁ generation: Progeny of primary transformants.

R₂ generation: Second generation progeny of Rₒ generation (i.e., progeny of R₁ generation).

A gene derived in part from a plant virus RNA molecule: At least the portion of the gene encoding the untranslatable RNA molecule is derived from a plant virus RNA molecule.

### GENERAL DESCRIPTION

An untranslatable plus sense RNA molecule is encoded by a gene located on the DNA molecule. The gene comprises DNA derived from a plant virus RNA genome and also DNA from heterologous sources. The DNA from heterologous sources includes elements controlling the expression of the virus-derived DNA sequences. The DNA sequence of the gene is specifically altered so as to render the RNA molecule transcribed from the gene untranslatable. The presence of this untranslatable plus sense RNA within the cells of the transformed plant reduces the susceptibility of the plant to viral infection.

More particularly, the portion of the gene which comprises DNA from a plant virus has been derived from a potyvirus. Plants transformed with the DNA molecule containing the gene are less susceptible to infection by potyviruses. Most specifically, the DNA from the potyvirus source has been derived from the coat protein gene of Tobacco Etch Virus and transformed plants are resistant to infection by Tobacco Etch Virus. Plants which can be made resistant to potyvirus infection include, but are not limited to, tobacco.

Accordingly, the present invention provides a method for genetically engineering plants by insertion, into the plant genome, a DNA construct containing a recombinant gene derived from a potyvirus genome such that the engineered plants display resistance to the potyvirus.

In accordance with one aspect of the presen invention, genetically transformed plants which are resistant to infection by a plant potyvirus are produced by inserting into the genome of the plant a DNA sequence which causes the production of an untranslatable coat protein RNA of the potyvirus.

In accordance with another aspect of the present invention, a DNA sequence is provided to function in plant cells to cause the production of an untranslatable plus sense RNA molecule. There has also been provided, in accordance with yet another aspect of the present invention, bacterial and transformed plant cells that contain the above-described DNA. In accordance with yet another aspect of the present invention, a differentiated tobacco plant has been provided that comprises transformed tobacco cells which express the untranslatable coat protein RNA of Tobacco Etch Virus and which plants exhibit resistance to infection by Tobacco Etch Virus.

A mechanism by which an untranslatable plus sense RNA molecule, such as described in the current invention can function to inhibit the normal biological function of a minus sense RNA molecule is proposed. One skilled in the art will recognize that the novel approach described herein is not limited to the specific experimental example given and will appreciate the wider potential utility of the invention.

The expression of a plant gene which exists in double-stranded DNA form involves transcription of messenger RNA (mRNA) from one strand of the DNA by RNA polymerase enzyme, and the subsequent processing of the mRNA primary transcript inside the nucleus. This processing involves a 3' nontranslated region which causes polyadenylate nucleotides to be added to the 3' end of the viral RNA. Transcription of DNA into mRNA is regulated by a region of DNA usually referred to as the "promoter." The promoter region contains a sequence of bases that signals RNA polymerase to associate with the DNA and to initiate the transcription of mRNA using one of the DNA strands as a template to make a corresponding strand of RNA.

A number of promoters which are active in plant cells have been described in the literature. Promoters which are known or are found to cause transcription of viral RNA in plant cells can be used in the present invention. Such promoters may be obtained from plants or viruses and include, but are not limited to, the CaMV 35S promoter. As described below, it is preferred that the particular promoter selected should be capable of causing sufficient expression to result in the production of an effective amount of untranslatable plus sense RNA to render the plant substantially resistant to virus infection. The amount of untranslatable plus sense RNA needed to induce resistance may vary with the plant type. Accordingly, while the 35S promoter is preferred, it should be understood that this promoter may not be the optimal one for all embodiments of the present invention. Furthermore, the promoters used in the DNA constructs of the invention may be modified, if desired, to affect their control characteristics. DNA sequences have been identified which confer regulatory specificity on promoter regions. For example, the small subunit of the ribulose bis-phosphate carboxylase (ss RUBISCO) gene is expressed in plant leaves but not in root tissues. A sequence motif that represses the expression of the ss RUBISCO gene in the absence of light, to create a promoter which is active in leaves but not in root tissue, has been identified. This and/or other regulatory sequence motifs may be ligated to promoters such as the CaMV 35S promoter to modify the expression patterns of a gene. Chimeric promoters so constructed may be used as described herein. For purposes of this description, the phrase "CaMV 35S promoter" will therefore include all promoters derived by means of ligation with operator regions, random or controlled mutagenesis, as well as tandem or multiple copies of enhancer elements, and the like.

The 3' nontranslated region of genes which are known or are found to function as polyadenylation sites for viral RNA in plant cells can be used in the present invention. Such 3' nontranslated regions include, but are not limited to, the 3' transcribed, nontranslated region of the CaMV 35S gene and the 3' transcribed, nontranslated regions containing the polyadenylation signals of the tumor-inducing (TI) genes of *Agrobacterium*, such as the tumor morphology large (tml) gene. For purposes of this description, the phrase "CaMV 35S 3' nontranslated region" will therefore include all such appropriate 3' nontranslated regions.

The DNA constructs of the disclosed embodiment contain, in double-stranded DNA form, a portion of a cDNA version of the single-stranded RNA genome of TEV. In potyviruses, including TEV, the viral genome includes genes encoding the coat protein, a replicase enzyme and a proteinase. The disclosed embodiment utilizes the region of the genome encoding the coat protein gene. In considering the present invention and the evidence for the proposed mechanism by which an untranslatable plus sense RNA molecule can inhibit viral replication, those skilled in the art will recognize that other portions of a potyvirus genome could be substituted for the coat protein gene. Furthermore, it will be apparent that suitable genomic portions are not limited to complete gene sequences.

A disclosed embodiment of the invention utilizes a double-stranded complementary DNA (cDNA) derived from the region of the TEV genome encoding the coat protein gene. To the 5' end of this cDNA is ligated the CaMV 35S promoter and CaMV 35S RNA 5' nontranslated region. To the 3' end is ligated the CaMV 35S 3' nontranslated region. These 5' and 3' sequences are present to cause transcription of the gene in plant cells by the cellular enzyme RNA polymerase to produce an RNA molecule of sequence corresponding to the sequence of the coat protein cDNA sequence. Ordinarily, such an RNA would then be translated by ribosomes which would synthesize a protein of amino acid sequence specified by the nucleotide sequence of the RNA molecule. Particular amino acids are specified by nucleotide triplets termed codons. Codons which stipulate translation initiation and termination are also present in DNA and RNA sequences. The current invention relates to RNA molecules which are untranslatable by ribosomes. In the preferred embodiment the sequence of the TEV cDNA encoding the coat protein is mutated by a standard *in vitro* mutagenesis technique to produce a frameshift mutation early in the coat protein structural gene immediately followed by three translation termination signal codons. These mutations do not affect the ability of RNA polymerase to transcribe an RNA molecule from the cDNA but prevent translation of the transcribed RNA by ribosomes. Those skilled in the art will recognize that for the disclosed gene and for other genes, DNA sequences can be altered in other ways to cause the DNA to encode an untranslatable plus sense RNA molecule. Thus the disclosed invention is not limited to the mutations disclosed.

A disclosed embodiment utilizes a cDNA encoding the coat protein gene of TEV, mutated so as to encode an untranslatable plus sense RNA. It will be obvious to one skilled in the art that further sequence alteration of the cDNA molecule could be used to confer additional features on the untranslatable plus sense RNA molecule. Additional features include those which would result in increased viral resistance of plants transformed with the cDNA molecule encoding an untranslatable plus sense RNA. The inclusion of a ribozyme sequence which causes the RNA catalyzed destruction of the target RNA molecule would constitute one such additional feature. Suitable ribozyme sequences are known, as discussed in Tabler and Tsagris (1991).

A DNA construct in accordance with the present invention is introduced, via a suitable vector and transformation method as described below, into plant cells and plants transformed with the introduced DNA are regenerated. Various methods exist for transforming plant cells and thereby generating transgenic plants. Methods which are known or are found to be suitable for creating stably transformed plants can be used in this invention. The choice of method will vary with the type of plant to be transformed; those skilled in the art will recognize the suitability of particular methods for given plant types. Suitable methods may include, but are not limited to: electroporation of plant protoplasts; liposome mediated transformation; polyethylene mediated transformation; transformation using viruses; microinjection of plant cells; microprojectile bombardment of plant cells and *Agrobacterium tumefaciens* (AT) mediated transformation. The latter technique is the method of choice for the disclosed preferred embodiment of the present invention.

In an embodiment of the current invention, the DNA sequences comprising the CaMV 35S promoter and CaMV 35S nontranslated 3' region and the mutated cDNA encoding an untranslatable plus sense RNA derived from the TEV coat protein gene are combined in a single cloning vector. This vector is subsequently transformed into AT cells and the resultant cells are used to transform cultured tobacco cells.

Vectors suitable for the AT mediated transformation of plants with the DNA of the invention are disclosed. It will be obvious to one skilled in the art that a range of suitable vectors is available, including those disclosed by Bevan (1983), Herrera-Estrella (1983), Klee (1985) and EP-A-120516 (Schilperoort et al.). Suitable vectors are available on a commercial basis from Clontech (Palo Alto, CA) and Pharmacia LKB (Pleasant Hill, CA) and other sources.

Following the transformation of plant cells and regeneration of transformed plants with the DNA molecules as described, regenerated plants are tested for increased virus resistance. Plants are preferably exposed to the virus at a concentration within a range where the rate of disease development correlates linearly with virus concentration. Methods for virus inoculation are well known to those skilled in the art and are reviewed by Kado and Agrawai (1972). One such method includes abrading a leaf surface with an aqueous suspension containing an abrasive material such as carborundrum and virus or dusting leaves with such an abrasive material and subsequently applying the virus onto the leaf surface. A virus suspension can be directly inoculated into leaf veins or alternatively plants can be inoculated using insect vectors. The virus suspension may comprise purified virus particles, or alternatively, sap from virus infected plants may be utilized.

Transformed plants are then assessed for resistance to the virus. The assessment of resistance or reduced susceptibility may be manifest in different ways dependant on the particular virus type and plant type. Those skilled in the art will realize that a comparison of symptom development on a number of inoculated untransformed plants with symptom development on similarly inoculated transformed plants will provide a preferred method of determining the effects of transformation with the specified DNA molecule on plant resistance. Symptoms of infection include, but are not limited to leaf mottling, chlorosis and etching. Plants showing increased viral resistance may be recognized by delay in appearance of such symptoms or attenuation or total lack of such symptoms.

### Example

Work with tobacco plants and the Tobacco Etch Virus (TEV) is illustrative of the invention.

### Construction of gene encoding untranslatable plus sense RNA molecule.

The Highly Aphid Transmissible (HAT) isolate of Tobacco Etch Virus (TEV) was obtained from Dr. Tom Pirone (University of Kentucky) and maintained in *Nicotiana tabacum* (Burley 21). The virus was purified from *Nicotiana tabacum* (Burley 21) 20 to 30 days following inoculation. Viral purification and RNA isolation procedures have been described (Dougherty and Hiebert (1980a). Complementary DNA (cDNA) was synthesized, made double-stranded and inserted into the bacterial plasmid pBR322 as described by Allison et al. (1985a, 1985b, 1986). cDNA synthesis was accomplished as follows: Purified viral RNA primed with oligo(dT₁₂₋₁₈) served as a template for single-strand cDNA synthesis by reverse transcriptase. Following the addition of homopolymeric tracts of deoxycytidine 5' monophosphate, second-strand synthesis, primed with oligo(dG₁₂₋₁₈), was completed with DNA polymerase I. *Sal*I and *Eco*RI linkers were ligated to the double-stranded cDNA and inserted into the bacterial plasmid pBR322 (Kurtz and Nicodemus 1981). The resulting cDNA clones were screened by colony hybridization (Hanahan and Meselson 1980) with oligo(dT₁₂₋₁₈) primed, ³²P-labeled single-stranded TEV cDNA. Plasmid DNA was isolated from colonies which hybridized with the probe, and the *Sal*I/*Eco*RI cDNA inserts were sized by electrophoresis in a 0.8% (w/v) agarose gel using a horizontal water-cooled gel apparatus.

The *Sal*I/*Eco*RI inserts from the recombinant molecules were isolated from an agarose gel with NA45 membrane (Schleicher & Schuell, Keene, NH) according to the manufacturer's protocol. The following restriction enzymes were used either alone or in combination to digest the isolated cDNA insert: *Hind*III, *Xho*I, *Alu*I, *Hae*III, *Rsa*I, *Sau*3A, and *Taq*I. Restriction enzyme digestion products were inserted into the DNA of an appropriate M13 bacteriophage (Messing 1983) seiecred for the presence of corresponding polylinker restriction sites, and their nucleotide sequences were determined by dideoxy chain termination.

Plasmid pTL 37/8595 (Carrington and Dougherty 1987; Carrington et al. 1987) contains a cDNA copy of the genomic sequence of HAT TEV corresponding to nucleotides (nt) 1-200 and nt 8462-9495 (Fig. 2). (Numbering of the TEV genome nucleotides is according to that presented in Allison et al. 1986). The nucleotide sequence and deduced amino acid sequence of the Tobacco Etch Virus genome and the numbering system utilized by Allison et al. (1986) and herein is shown in Fig. 1 and SEQ ID No. 1 in the attached sequence listing. The first and last codons of the coat protein (CP) coding region in the TEV genome are nt 8518-8520 (encoding the amino acid serine) and 9307-9309 (opal stop codon) respectively. pTL 37/8595 was subject to *in vitro* site-directed mutagenesis as described by Taylor et al. (1985a, 1985b). In all cases, nucleotide changes were confirmed by dideoxy-nucleotide sequencing (Sanger et al. 1977).

TEV nt 9312-9317 were first mutated (Fig. 2) to generate a *Bam*HI restriction site (GGATCC). TEV nt 8516-8521 were then altered to generate an *Nco*I site (CCATGG), changing the first codon of the TEV CP coding region from AGT (Ser), to ATG (Met). A single oligonucleotide was then used to mutate TEV nt 133-138 to a *Bam*HI restriction site (GGATCC), nt 143-148 to an NcoI restriction site (CCATGG) and nt 142 to a deoxyadenylate residue. These mutations generated an NcoI site centered on the first codon of the TEV ORF and in a good translational start context as described by Kozak (1984). Digestion of the resulting plasmid with the restriction enzyme *Nco*I; removing TEV nt # 143-200/8462-8516, and religation generated plasmid pTC:FL. pTC:FL contained only the TEV CP gene flanked by *Bam*HI restriction sites and TEV 5' and 3' untranslated sequences (see Fig. 2). The nucleotide sequence of the TEV CP gene in pTC:FL produced by this mutagenesis scheme is shown in SEQ ID No. 2 in the attached sequence listing.

Plasmid pTC:RC (RNA Control, producing untranslatable plus sense RNA) was generated by insertion of a single deoxythymidylate residue after TEV nt 8529, and point mutations of TEV nt 8522 (G to C), 8534 (C to A), 8542 (G to A), and 8543 (A to G) to create a frameshift mutation immediately followed by three stop codons. An *Nhe*I restriction site (GCTAGC) was simultaneously generated, for screening purposes, at nt 8539-8544. The nucleotide sequence of the TEV CP gene in pTC:RC produced by this mutagenesis scheme is shown in SEQ ID No. 3 in the attached sequence listing.

All plasmids described above were linearized with *Hind*III, transcribed with T7 RNA polymerase (Melton et al. 1984), and translated in a rabbit reticulocyte lysate containing ³⁵S Methionine (Dougherty and Hiebert 1980a). Radiolabeled translation products were analyzed by electrophoretic separation on a 12.5% acrylamide gel containing SDS (Laemmli 1970) and detected by autoradiography. Transcripts of plasmid pTC:RC produced no detectable protein products, while transcripts from pTC:FL produced proteins of the expected sizes.

The various forms of the CP nucleotide sequence were then inserted as *Bam*HI cassettes into the plant expression vector pPEV (see below and Fig. 3).

The full length TEV CP open reading frame of pTC:FL was inserted in the reverse orientation to make the antisense (AS) construct pTC:AS. The nucleotide sequence of the TEV CP gene in pTC:AS is shown in SEQ ID No. 4 in the attached sequence listing.

### Transformation Vector Construction

Construction of pPEV. The vector pPEV is part of a binary vector system for *Agrobacterium tumefaciens* mediated plant cell transformation. Plasmid pPEV was constructed from the plasmids pCGN 2113 (Calgene), pCIB 710 and pCIB 200 (Ciba Geigy Corp.). pCGN 2113 contains the "enhanced" Cauliflower Mosaic Virus (CaMV) 35S promoter (CaMV sequences -941 to 90/-363 to +2, relative to the transcription start site) in a pUC derived plasmid backbone. pCIB 710 has been described (Rothstein et al. 1987) and pCIB 200 is a derivative of the wide host range plasmid pTJS 75 (Schmidhauser and Helinski 1985) which contains left and right *A. tumefaciens* T37 DNA borders, the plant selectable NOS/NPT II chimeric gene from the plasmid Bin 6 (Bevan 1984) and part of a pUC polylinker. The small *Eco*RI-*Eco*RV DNA fragment of pCIB 710 (Rothstein et al. 1987) was ligated into *Eco*RI*-Eco*RV digested pCGN 2113. This regenerated the enhanced CaMV 35S promoter (Kay et al. 1987) of pCGN 2113 and introduced the CaMV 35S 5' and 3' untranslated sequences into pCGN 2113. The CaMV 35S promoterterminator cassette of the resulting plasmid was isolated as an *Eco*RI-*Xba*I DNA fragment and ligated into *Eco*RI-*Xba*I digested pCIB 200 to generate pPEV. CP nucleotide sequences from PTC:FL, pTC:RC, and pTC:AS were cloned as *Bam*HI cassettes into *Bam*HI digested pPEV and orientation of inserts confirmed by digestion with appropriate restriction endonucleases.

### Transformation and Regeneration of Tobacco

pPEV plasmids containing TEV CP ORFs were mobilized from *E*. *coli* HB101 into *A*. *tumefaciens* A136 containing plasmid pCIB 542 (Ciba Geigy), using the helper plasmid pRK 2013 in *E. coli* HB101 and the tri-parental mating system of Ditta et al. (1980). Plasmid pCIB 42 supplied *vir* functions necessary for T-DNA transfer.

Leaf discs of *Nicotiana tabacum cv* Burley 49 were transformed and whole plants regenerated according to Horsch et al. (1985). Transformed tissue was selected by culturing callus on MS plates (Murashige and Skoog 1962) containing 1 *µ* g/ml 6-benzylaminopurine (Sigma Corp.), 01 *µ*g/ml α-naphthaleneacetic acid (Sigma Corp.), 500 *µ*g/ml carbenicillin and 100 *µ*g/ml Kanamycin sulfate (Sigma Corp.). Shoots were rooted on MS plates containing 500 *µ*g/ml carbenicillin and 100 *µ*g/ml kanamycin sulfate, and plantlets were transplanted into soil and transferred directly into the greenhouse approximately 2-3 weeks after rooting.

R0, R1 and R2 generation plants were screened by western and/or northern blot analyses. R2 seed (ca. 100 seeds per R2 plant) was screened for the kanamycin-resistant phenotype (kan^{r}) by surface sterilizing seed in 10% bleach for 5 min., washing twice in sterile water and germinating on MS plates containing 100 *µ*g/ml kanamycin sulfate. R2 seed lines which were 100% kanamycin resistant were screened by western blot analysis for expression of TEV coat protein. Those transgenic plant lines generated and their nomenclature are presented in Fig. 3.

### Molecular Analyses of Transgenic Plants

Transgenic tobacco plants were analyzed by western and northern blot analyses to determine the nature of protein and RNA products produced respectively. Total RNA samples isolated from the various transgenic lines were analyzed in northern blot hybridization studies. Total nucleic acids were isolated from tissue and RNA precipitated with LiCl as described by Verwoerd et al. (1989). RNAs were electrophoretically separated on 1.2% agarose gels containing 6% (v/v) formaldehyde and transferred to nitrocellulose. Prehybridization and hybridization conditions were as described in Sambrook et al. (1989). Strand specific riboprobes were generated from SP6 or T7 DNA dependent RNA polymerase transcription reactions of pTL 37/8595 linearized with the restriction enzymes Asp718 (Boehringer Mannheim, Indianapolis, IN) or *Hind*III, respectively, using α-labelled ³²P-CTP ribonucleotide and suggested procedures (Promega, Madison, WI).

An RNA transcript of approximately 1,000 nt was expected with all transgenic plant lines. Such a TEV CP transcript was detected in CP expressing plant lines by using a minus sense riboprobe containing the TEV CP sequence. A similar transcript was detected in AS plants by using a plus sense riboprobe containing the TEV CP sequence. The transcript in the RC line, while detected with a minus sense riboprobe, may have migrated as a slightly larger (ca 1,100-1,200 nt) RNA species, possibly due to termination at an alternately selected site and/or a longer poly-A tail on the transcript. Differing levels of CP transcript accumulation were observed among different transgenic plant lines. Transgenic plant lines expressing the coat protein of TEV were identified by western blot analysis using polyclonal antisera to TEV CP. Tissue samples of regenerated plants were ground in 10 volumes of 2X Laemmli (Tris-glicine) runner buffer (Laemmli 1970) and clarified by centrifugation in a microcentrifuge for 10 min. at 10,000xg. Protein concentration was estimated by the dye binding procedure of Bradford (1976) using BSA as a standard. Protein samples (50 *µ*g total protein) were separated on a 12.5% polyacrylamide gel containing SDS and subjected to the immunoblot transfer procedures described by Towbin et al. (1979). Anti-TEV coat protein polyclonal primary antibodies, alkaline phosphatase conjugated secondary antibodies and the chromogenic substrates NBT (para-nitro blue tetrazolium chloride) and BCIP (5-bromo-4-chloro-3-indoyl phosphate para-toluidine salt) were used to detect bound antigen.

Coat protein products produced in FL plants were stable and accumulated to different levels in individual transgenic plant lines. It was estimated by western blot analysis that between 0.01% to 0.001% of total extracted protein was TEV CP.

### Assessment of Resistance to TEV

Eight-week-old (circa 15 cm tall) R1 and R2 plants were inoculated with either purified virus preparations or infected plant sap. Inoculum was applied with sterile, premoistened cotton swabs. Infected plant sap inoculum was prepared by grinding TEV-infected *N. tabacum* Burley 21 leaf tissue (2 weeks postinoculation) in carborundum and 50 mM sodium phosphate buffer (pH 7.8) at a ratio of 1gm:02gm:10mls, respectively, and filtering the homogenate through cheesecloth. TEV virons were purified as described by Dougherty and Hiebert (1980b). One leaf per plant was dusted lightly with carborundum (320 grit) and inoculated at two interveinal locations with 50 *µ*l (total) of inoculum. Inoculated plants were examined daily and the appearance and severity of systemic symptoms recorded. Symptoms on any leaf above the inoculated leaf were considered to be systemic.

Typically, inoculation of Burley 49 plants with TEV (either purified virus or plant sap) resulted in severe chlorosis and mosaic and mottle on systemically infected leaves approximately 6-7 days after inoculation. Severe etching of the leaf followed within a few days. It was observed that transgenic plants containing only the CaMV promoter and untranslated sequences (i.e., 35S plant line) responded to challenge inoculation in a manner similar to wild type Burley 49, developing extensive chlorosis and etching at the same rate (Fig. 4A). Plant lines which expressed FL TEV CP showed little or no delay in the appearance of symptoms when inoculated with infected plant sap. However, FL transgenic plants did show a slight attenuation of symptoms and eventually (2-4 weeks after initial appearance of symptoms), younger leaf tissue emerged devoid of symptoms and virus as demonstrated by back inoculation experiments. Typically chlorosis and etching on older systemic leaves was limited.

Ten independently transformed RC lines and seven independently transformed AS lines were obtained. Progeny from three of the RC lines, including line RC #5 and from one of the AS lines, including AS #3, showed an altered response to viral infection relative to control plants. All of these lines were verified to be transformed and were producing expected RNA products. A possible explanation for the variation in observed phenotype is the previously noted "position effect" whereby the expression of genes from identical DNA sequences integrated at different locations within the genome show varying patterns of tissue specificity.

Ten R2 expressing plants of the FL expressing line were inoculated with infected plant sap, and 20 R1 plants of lines AS #3 and RC #5 were inoculated with 50 *µ*l of a 5 *µ*g/ml solution of purified TEV. Identical results to those obtained by purified TEV inoculation were obtained when AS #3 and RC #5 R1 plants were inoculated with TEV-infected plant sap, as described above.

Transgenic Burley 49 plant lines AS #3 and RC #5, expressing only TEV CP related RNA sequences, showed a delay in the appearance of symptoms and a modification of symptoms when inoculated with TEV (Fig. 4B). Since the 20 R1 plants were not screened for expression of CP RNA prior to inoculation, some of the symptomatic plants represented non-expressing plants in which the gene of interest had been lost during Mendelian segregation. Modified symptoms on AS #3 plants appeared as small chlorotic lesions often associated with a vein. Most of the leaves were devoid of symptoms and virus (determined by back inoculation experiments). Approximately 15% of RC #5 plants showed symptoms which were identical to those of infected Burley 49. However, the remaining RC #5 plants were entirely asymptomatic, and virus was not detected in back inoculation studies.

Plants from TEV resistant AS and RC lines showed no increased resistance, relative to untransformed controls, to infection by two other members of the potyvirus family, namely Tobacco Vein Mottling Virus and Potato Virus Y.

R₂ generation plants derived from TEV-resistant RC plants showed the expected Mendelian pattern of inheritance of the TEV-resistant phenotype.

### Analysis of TEV Replication in Protoplasts Derived from Transgenic Plant Lines

In an attempt to explain the results obtained when AS and RC transgenic plants were challenged with TEV, it was sought to determine if all of the transgenic plant lines would support virus replication at a level comparable to Burley 49. Accumulation of viral encoded proteins was used as an indirect indicator of viral replication. Protoplasts were derived from leaf tissue of homozygous CP expressing plants and electroporated according to the procedure of Luciano et al. (1987) with TEV RNA. Protoplasts were prepared from transgenic plants and electroporated according to the procedure of Luciano et al. (1987). Protoplasts (1 X 10⁶) were resuspended in 450 *µ*l electroporation buffer (330 mM mannitol, 1 mM KPO₄ pH 7.0, 150 mM KCl) and electroporated using a BTX Transfector 300 (BTX San Diego, CA) (950 micro Farads, 130-volt pulse amplitude, 3.5 mm electrode gap) in the presence or absence of 6 *µ*g of purified TEV RNA. After electroporation, protoplasts were incubated for 96 hours in incubation medium as described in Luciano et al. (1987). Protoplasts were extracted in 2X Laemmli (Trisglycine) running buffer, and 5 x 10⁴ extracted protoplasts were then subjected to western blot analysis as described above. Protoplast viability was measured by dye exclusion as described in Luciano et al. (1987). All electroporated protoplast samples had equivalent viability counts. The results indicated that protoplasts from all FL plant lines supported virus replication at levels comparable to wild type Burley 49 protoplasts. R1 transgenic plants from lines AS #3 and RC #5 were initially screened by northern analysis, and leaves from positive expressors were used in the production of protoplasts. Transfected protoplasts derived from AS #3 plants supported TEV replication, albeit at a reduced level. Protoplasts derived from RC #5 transgenic plant leaf tissue did not support TEV replication at a detectable level. These results, and those presented in the whole plant inoculation series, suggested AS and RC plants interfere with TEV replication.

### Discussion of Data

The above example indicates that varying degrees of protection from TEV infection can be achieved by overexpression of coat protein and by expression of an antisense RNA. The current invention which comprises the expression of an untranslatable plus sense RNA molecule provides protection against TEV infection that is more effective than either of these two methods. Plants of line RC #5, transformed with the disclosed DNA molecule encoding an untranslatable plus sense RNA derived from the TEV coat protein gene, were asymptomatic and appear to be completely protected from virus infection. The disclosed invention therefore represents a new and effective way of generating potyvirus resistant germplasm.

Tobacco protoplasts derived from plants expressing the antisense RNA supported a reduced level of TEV replication compared to control cells derived from untransformed plants. In contrast, tobacco protoplasts derived from plants of line RC #5, expressing the untranslatable plus sense RNA did not support detectable TEV replication. This suggests that the untranslatable plus sense RNA was more effective at blocking TEV replication in the cells of those transformed plants tested.

It is proposed that the untranslatable plus sense RNA inhibits viral replication by hybridizing to the minus sense RNA replicative template of TEV. The finding that plants expressing untranslatable plus sense RNA derived from the TEV coat protein gene are not protected from infection by Potato Virus Y or Tobacco Vein Mottling Virus is therefore explained by the circa 40-50% amino acid sequence divergence between the coat proteins of these viruses and TEV (Allison et al. 1986; Robaglia et al. 1989; Domier et al. 1986).

From the above-described findings, it would be reasonable and entirely predictable that if plants were transformed with a gene encoding an untranslatable plus sense RNA derived from a gene which was highly conserved between viruses of the potyvirus family, that these plants would be protected from infection by a wide range of viruses. Regions of the potyvirus genome which are sufficiently conserved between potyvirus types to be potentially useful in such an approach may be readily determined by one skilled in the art. Highly conserved regions may be determined by reference to published sequence data (Allison et al. 1986; Robaglia et al. 1989; Domier et al. 1986; Lain et al. 1989; Maiss et al. 1989). The utility of the identified regions could be readily determined using the methodologies described above and substituting the defined region for the TEV coat protein gene.

Regions of the potyvirus genome potentially suitable include, but are not limited to the genes encoding the viral replicase and the viral proteinase. Furthermore, it will be apparent to one skilled in the art that highly conserved portions of a particular gene may also serve in this role.

It will also be apparent to one skilled in the art that the described invention may also be used to produce plants resistant to viruses outside of the potyvirus family in instances where these viruses also produce a minus sense RNA replicative template.

### BIBLIOGRAPHY

Allison et al. 1985a. Virology 147:309-316. Allison et al. 1985b. Proc. Natl. Acad. Sci. USA 82:3969-3972.

Allison et al. 1986. Virology 154:9-20.

Bevan 1984. Nucl. Acids Res. 12:8711-8721.

Bradford 1976. Nucl. Acids Res. 12:8711-8721.

Carrington and Dougherty 1987. J. Virol. 61:2540-2548.

Carrington et al. 1987. Nucl. Acids Res. 15:10066.

Ditta et al. 1980. Proc. Natl. Acad. Sci. USA 77:7347-7351.

Domier et al. 1986. Nucl. Acids Res. 14:5417-5430.

Dougherty and Hiebert 1980a. Virology 101:466-474.

Dougherty and Hiebert 1980b. Virology 104:183-194.

Hanahan and Meselson 1980. Gene 10:63-67.

Herrera-Estrella et al. 1983. Nature (London) 303:209-213.

Hollings and Brunt 1981. Commonwealth Mycological Institute/Association of Applied Biologists Descrip. Plant Viruses, No. 245.

Horsch et al. 1985. Science 227:1229-1231.

Kado and Agrawai 1972. Principles and Techniques in Plant Virology.

Kay et al. 1987. Science 236:1299-1302.

Kozak 1984. Nature (London) 308:241-246.

Kurtz and Nicodemus 1981. Gene 13:145-152.

Laemmli 1970. Nature (London) 227:680-685.

Lain et al. 1989. Virus Res. 13:157-172.

Lawson et al. 1990. Bio/Technology 8:127-134.

Ling et al. 1991. Bio/Technology 9:752-758.

Luciano et al. 1987. Plant Science 51:295-303.

Maiss et al. 1989. J. Gen. Virol. 70:513-524.

Melton et al. 1984. Nucl. Acids Res. 12:7145-7156.

Messing 1983. "Methods in Enzymology" (R. Wu, L. Grossman, and K. Moldave, eds.). Vol. 101c, pp. 20-78. Academic Press, New York.

Murashige and Skoog 1962. Plant Physiol. 15:473-497.

Robaglia et al. 1989. J. Gen. Virol. 70:935-947.

Rothstein et al. 1987. Gene 53:153-161.

Sambrook et al. 1989. Molecular cloning: a laboratory manual, 2nd ed. Cold Spring Harbor Laboratories, Cold Spring Harbor, New York.

Sanford and Johnston 1985. J. Theor. Biol. 113:395-405.

Sanger et al. 1977. Proc. Natl. Acad. Sci. USA 74:5463-5467.

Schmidhauser and Helinski 1985. J. Bact. 164:446-455.

Stark and Beachy 1989. Bio/Technology 7:1257-1262.

Tabler and Tsagris 1991. Gene 100:175-183.

Taylor et al. 1985a. Nucl. Acids Res. 13:8749-8764.

Taylor et al. 1985b. Nucl. Acids Res. 13:8765-8785.

Towbin et al. 1979. Proc. Natl. Acad. Sci. USA 76:4350-4354.

Verwoerd et al. 1989. Nucl. Acids Res. 17:2372.

## Claims

1. A method of producing a plant with a reduced susceptibility to viral infection, comprising:
transforming plant cells with a DNA molecule that encodes untranslatable plus-sense viral RNA molecule wherein the untranslatable plus-sense viral RNA molecule is derived from the nucleotide sequence of a plant virus gene; and
regenerating a plant comprising the transformed plant cell.

2. A transgenic plant produced according to the method of Claim 1.

3. The method of Claim 1 wherein the untranslatable plus-sense viral RNA molecule is derived from a viral coat protein gene.

4. The method of Claim 1 wherein the untranslatable plus-sense viral RNA molecule is derived from a potyvirus.

5. A DNA molecule useful for producing virus resistant plants comprising a promoter operably linked to a DNA molecule encoding an untranslatable plus-sense viral RNA molecule, derived from the nucleotide sequence of a plant virus gene.

6. The method of any one of Claims 1, 3 or 4 wherein the untranslatable plus-sense viral RNA molecule contains at least one mutation that renders the RNA molecule untranslatable, and expression of the untranslatable plus-sense viral RNA molecule within the plant reduces the susceptibility of the plant to virus infection;
and wherein the method further comprises the step of selecting a plant that shows a reduced susceptibility to infection by the virus.

## Patentansprüche

1. Ein Verfahren zur Herstellung einer Pflanze mit einer reduzierten Anfälligkeit für eine virale Infektion, umfassend:
Transformation von Pflanzenzellen mit einem DNA-Molekül, das für ein nicht-translatierbares plus-strang virales RNA-Molekül kodiert, **dadurch gekennzeichnet, daß** das nicht-translatierbare plus-strang virale RNA-Molekül von der Nukleotidsequenz eines Pflanzenvirusgens abgeleitet ist; und
Regeneration einer Pflanze, beinhaltend die transformierte Pflanzenzelle.

2. Eine transgene Pflanze, hergestellt entsprechend des Verfahrens nach Anspruch 1.

3. Das Verfahren nach Anspruch 1, **dadurch gekennzeichnet, daß** das nicht-translatierbare plus-strang virale RNA-Molekül von einem viralen Hüllproteingen abgeleitet ist.

4. Das Verfahren nach Anspruch 1, **dadurch gekennzeichnet, daß** das nicht-translatierbare plus-strang virale RNA-Molekül von einem Potyvirus abgeleitet ist.

5. Ein DNA-Molekül, verwendbar zur Herstellung virusresistenter Planzen, umfassend einen Promoter, wirksam verbunden mit einem DNA-Molekül, das für ein nicht-translatierbares plus-strang virales RNA-Molekül kodiert, welches von der Nukleotidsequenz eines Pflanzenvirusgens abgeleitet ist.

6. Das Verfahren nach einem der Ansprüche 1, 3 oder 4, **dadurch gekennzeichnet, daß** das nicht-translatierbare plus-strang virale RNA-Molekül mindestens eine Mutation enthält, die das RNA-Molekül nicht-translatierbar macht, und daß die Expression des nicht-translatierbaren plus-strang viralen RNA-Moleküls in der Pflanze zu einer reduzierten Anfälligkeit der Pflanze gegen Virusinfektion führt;
und, **dadurch gekennzeichnet, daß** das Verfahren weiterhin den Schritt der Selektion einer Pflanze umfaßt, die eine reduzierte Anfälligkeit für eine Infektion durch das Virus zeigt.

## Revendications

1. Méthode de production d'une plante avec une sensibilité réduite à l'infection virale, comprenant les étapes consistant :
à transformer des cellules végétales avec une molécule d'ADN qui code une molécule d'ARN viral sens plus non traduisible, la molécule d'ARN viral sens plus non traduisible étant dérivée de la séquence nucléotidique d'un gène de virus de plante ; et
à régénérer une plante comprenant la cellule végétale transformée.

2. Plante transgénique produite selon la méthode de la revendication 1.

3. Méthode selon la revendication 1, dans laquelle la molécule d'ARN viral sens plus non traduisible est dérivée d'un gène de protéine de capside virale.

4. Méthode selon la revendication 1, dans laquelle la molécule d'ARN viral sens plus non traduisible est dérivée d'un potyvirus.

5. Molécule d'ADN utile pour produire des plantes résistantes à des virus, comprenant un promoteur lié de façon opérationnelle à une molécule d'ADN codant une molécule d'ARN viral sens plus non traduisible, dérivée de la séquence nucléotidique d'un gène de virus de plante.

6. Méthode selon l'une quelconque des revendications 1, 3 ou 4, dans laquelle la molécule d'ARN viral sens plus non traduisible contient au moins une mutation qui rend la molécule d'ARN non traduisible, et l'expression de la molécule d'ARN viral sens plus non traduisible dans la plante réduit la sensibilité de la plante à une infection virale ;
et la méthode comprenant en outre l'étape consistant à sélectionner une plante qui montre une sensibilité réduite à une infection par le virus.
